(19) Europäisches Patentamt
European Patent Office
Office européen des brevets

(11) **EP 1 040 349 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**19.12.2012   Patentblatt 2012/51**

(45) Hinweis auf die Patenterteilung:
**05.09.2001   Patentblatt 2001/36**

(21) Anmeldenummer: **98932469.4**

(22) Anmeldetag: **28.07.1998**

(51) Int Cl.:
**G01N 33/487** (2006.01)          **C12M 1/34** (2006.01)
**G01N 33/483** (2006.01)

(86) Internationale Anmeldenummer:
**PCT/IB1998/001150**

(87) Internationale Veröffentlichungsnummer:
**WO 1999/031503 (24.06.1999 Gazette 1999/25)**

(54) **POSITIONIERUNG UND ELEKTROPHYSIOLOGISCHE CHARAKTERISIERUNG EINZELNER ZELLEN UND REKONSTITUIERTER MEMBRANSYSTEME AUF MIKROSTRUKTURIERTEN TRÄGERN**

POSITIONING AND ELECTROPHYSIOLOGICAL CHARACTERIZATION OF INDIVIDUAL CELLS AND RECONSTITUTED MEMBRANE SYSTEMS ON MICROSTRUCTURED CARRIERS

POSITIONNEMENT ET CARACTERISATION ELECTROPHYSIOLOGIQUE DE CELLULES INDIVIDUELLES ET DE SYSTEMES MEMBRANAIRES RECONSTITUES SUR DES SUPPORTS MICROSTRUCTURES

(84) Benannte Vertragsstaaten:
**AT BE CH DE DK ES FI FR GB GR IE IT LI LU NL PT SE**

(30) Priorität: **17.12.1997   CH 290397**

(43) Veröffentlichungstag der Anmeldung:
**04.10.2000   Patentblatt 2000/40**

(73) Patentinhaber:
• **ECOLE POLYTECHNIQUE FEDERALE DE LAUSANNE (EPFL)**
  **1015 Lausanne (CH)**
• **MOLECULAR DEVICES CORPORATION**
  **Sunnyvale, CA 94089 (US)**

(72) Erfinder:
• **Vogel, Horst**
  **CH-1028 Préverenges (CH)**
• **Schmidt, Christian**
  **CH-1024 Ecublens (CH)**

(74) Vertreter: **Maiwald Patentanwalts GmbH**
**Elisenhof**
**Elisenstrasse 3**
**80335 München (DE)**

(56) Entgegenhaltungen:
EP-A- 0 094 193          WO-A-85/02201
FR-A- 2 659 347          US-A- 4 055 799
US-A- 4 894 343

**Beschreibung**

Verwandte Anmeldungen

Diese Anmeldung beansprucht die Priorität der Schweizer Anmeldung Nr. 2903/97, die am 17. Dezember 1997 hinterlegt wurde.

Technisches Gebiet

Die vorliegende Erfindung betrifft eine durch die Erfindung ermöglichte Messanordnung sowie ein Positionierverfahren für Zellen und Vesikel resp. Lipidmembranen, das Untersuchungen an Membranen ermöglicht, insbesondere ein elektrophysiologisches Verfahren zur Untersuchung von kanalbildenden Proteinen und mittels kanalbildender Proteine oder an kanalbildende Proteine gekoppelten Rezeptoren über die gemessene elektrische Eigenschaft der kanalbildenden Proteine. Insbesondere betrifft das erfindungsgemässe Messverfahren ein (Multiarray-)Patch-clamp Verfahren, welches die Empfindlichkeit und Selektivität der klassischen Patch-clamp-Technik besitzt, gleichzeitig aber infolge des ebenfalls erfindungsgemässen Verfahrens der Positionierung von biologischen Zellen oder Vesikeln auf mikrostrukturierten Trägern eine einfachere Herstellung der Patch-Membranen sowie hohen Signal-Rauschabstand gestattet. Ferner betrifft die vorliegende Erfindung eine Messanordnung, die sowohl für die Positionierung als auch für die elektrophysiologische Messung geeignet ist.

Stand der Technik

Viele biologisch wichtige Signaltransduktionsprozesse wie z.B. Nervenleitung, ereignen sich auf oder in Zellmembranen. Es ist deshalb nicht überraschend, dass die biologischen Funktionen von Membranproteinen im allgemeinen, und von Neurorezeptoren im besonderen durch pharmakologisch aktive Wirkstoffe beeinflusst werden (J.-P. Changeux (1993). "Chemical signalling in the brain". Sci. Am. Nov. Seiten 30 ff; A.G. Gilman (1995). Angew. Chem. Int. Ed. Engl. 34: 1406-1428; M. Rodbell (1995). Angew. Chem. Int. Ed. Engl. 34: 1420-1428).

Das funktionelle Verständnis der molekularen Wechselwirkungen an Rezeptoren sowie der Einsatz solcher Rezeptoren beim Wirkstoffscreening spielen in der modernen Arzneimittelentwicklung eine zentrale Rolle. Mit der steigenden Zahl der bekannten Zielrezeptoren (auch Target-Rezeptoren genannt) für Wirkstoffe und der schnell anwachsenden Zahl von potentiellen Wirkstoffen aus der kombinatorischen Chemie, wächst der Bedarf an hochempfindlichen Screeningmethoden, die es erlauben eine grosse Zahl von verschiedenen Substanzen bei hohem zeitlichem Durchsatz zu analysieren ("high trough-put screening" = HTS).

Gegenwärtig folgt man beim pharmakologischen Wirkstoffscreening noch relativ traditionellen Wegen, indem zeitaufwendige Ligandenbindungstests und Rezeptorfunktionstests getrennt durchgeführt werden (J. Hodgson (1992) Bio/Technology 9: 973). Andererseits zählen Membranproteine wie die an G-Proteine gekoppelten Rezeptoren und die kanalbildenden Rezeptoren zu den wichtigsten Zielproteinen für aktive Wirkstoffe (J. Knowles (1997) "Medicines for the new millennium hunting down deseases" Odyssey Vol. 3 (1)). In diesem Zusammenhang werden immer noch klassische Patch-clamp Methoden als funktionelle Rezeptortests eingesetzt. Der Vorteil dieser elektrophysiologischen Methode liegt darin, dass die Funktion der entsprechenden kanalbildenden oder an kanalbildende Proteine gekoppelten Rezeptoren über die gemessenen elektrischen Eigenschaften der kanalbildenden Proteine direkt zugänglich ist. Die Methode ist hochspezifisch und extrem empfindlich - man kann im Prinzip die Kanalaktivität einzelner Rezeptormoleküle messen. Dabei werden Glasmikropipetten mit einem Öffnungsdurchmesser von typischerweise 1-0,1 $\mu$m auf die Oberfläche einer biologischen Zelle aufgesetzt. Die Membranfläche, die von der Mikropipette abgedeckt wird, nennt man "Patch". Wenn der Kontakt zwischen der Glaselektrode und der Zellmembranoberfläche elektrisch hinreichend isolierend ist, kann mit Hilfe von Mikroelektroden, die einerseits in der Glaspipette und andererseits im membrangegenüberliegenden Milieu plaziert sind, der Ionenstrom über den Membranpatch elektrisch gemessen werden (O.P. Hamill, A. Marty, et al. (1981). "Improved patch-clamp techniques for high-resolution current recording from cells and cell-free membrane patches." Pflugers Arch 391(2): 85-100).

Im Zusammenhang mit Wirkstoffscreening weist die traditionelle Patch-clamp Technik allerdings auch entscheidende Nachteile auf. Patch-clamp Messungen sind äusserst zeitaufwendig, erfordern speziell geschultes Personal mit langen Erfahrungen auf diesem Gebiet und sind für HTS praktisch nicht einsetzbar.

Aus US-A-4 055 799 sind eine Methode und ein Verfahren zur Messung der elastischen und dielektrischen Eigenschaften des Diaphragmas lebender Zellen bekannt. Die offengelegte Vorrichtung ist ein Behälter, der zwei Messelektroden für Spannungsunterschiede, 2 Elektroden für die Aussendung von Spannung und Stromimpulsen, eine Trennwand, die den Behälter in 2 Kammern unterteilt und die ein oder mehrere Löcher enthält, einen Anschluss für physiologische Lösung und einen Anschluss für die Zufuhr von Elektrolytlösung. Für die Messung befinden sich die Zellen teilweise im Loch der Trennwand, so dass keine planare Membran über der Öffnung ausgebildet wird. Auch EP-A-0 094 193 und WO-A-8 502 201 offenbaren die Fixierung von Zellen in Löchern eines Trägers, wobei die Fixierung durch elektrisches Laden des Trägers erfolgt, was zu keiner inherent genauen Positionierung von Zellen oder Vesikeln über einem vorher definierten Punkt (der Apertur) mit einem Durchmesser kleiner als der Durchmesser dieser Objekte führen kann.

Ziel der vorliegenden Erfindung war es deshalb ein Mess- und Positionierverfahren bereitzustellen, das einfach in der Handhabung ist und rasche Untersuchung erlaubt, insbesondere für eine (Multiarray-)Patch-Clamp Methode, welche die Empfindlichkeit und Selektivität der klassischen Patch-Clamp Technik besitzt, gleichzeitig aber deren Nachteile infolge der erfindungsgemässen Methode der automatischen Positionierung von biologischen Zellen oder Vesikeln resp. entsprechender Lipidmembranen sowie der spezifischen Oberflächeneigenschaften der Messanordnung eliminiert. Ferner wird durch die vorliegende Erfindung eine planare Positionier- und Messanordnung ermöglicht, die für die Ausführung der erfindungsgemässen Verfahren speziell geeignet ist.

Darstellung der Erfindung

Die erfindungsgemässen Verfahren zeichnen sich aus durch äusserste Einfachheit in der Herstellung elektrisch abdichtender Patchmembranen sowie bei der anschliessenden Messung; in Kombination mit modernen mikrotechnologischen Methoden bietet die neue Technologie alle Möglichkeiten für den Einsatz im "high trough-put screening" (HTS). Ausserdem eignen sich die erfindungsgemässe Positionier- resp. Messanordnung sowie die erfindungsgemässen Verfahren zur Kombination von elektrischen und optischen Messungen, durch die an diesen mittels des erfindungsgemässen Positionierverfahrens erhaltenen planaren Membranen neue wichtige Informationen über die zu untersuchenden Rezeptoren erhalten werden können.

Das erfindungsgemässe Positionierverfahren für Zellen und Vesikel, resp. entsprechender Lipidmembranen zeichnet sich dadurch aus, dass zwischen zwei Elektroden eine Trennwand aus elektrisch isolierendem Material, in der Folge als Träger bezeichnet, angeordnet ist. Der Träger weist eine Apertur auf sowie eine Oberfläche, an der die Membranen fixiert werden. Der Träger muss nicht aus einem Stück bestehen, sondern er kann z.B. aufgebaut sein aus einem Halter, auf dem das für die Membranbindung und Membranpositionierung eigentlich relevante Material befestigt oder in den dieses Material eingelassen ist, wobei dieses Material für die Bindung resp. Positionierung der Membranen mindestens eine Apertur aufweist. Die Fixierung der Membranen kann z.B. auf elektrostatischen Wechselwirkungen zwischen einer beispielsweise negativ geladenen Membranoberfläche und einer positiv geladenen Trägeroberfläche beruhen. Falls die Trägeroberfläche als solche nicht die gewünschte Ladung aufweist, so kann sie entsprechend modifiziert werden. Es hat sich gezeigt, dass Zellen und Vesikel sehr gut positionierbar sind, wenn sie als Suspension über eine Zugabeöffnung von üblicherweise 0,2 - 2 mm Durchmesser, bevorzugt 0,5 - 1 mm in der einen Elektrode oder über einen nahe der Apertur angebrachten Schlauch oder mittels einer Pipette in. die Apparatur gegeben werden, wobei die beiden jeweils oberhalb und unterhalb des Trägers angeordneten Elektroden eine solche elektrische Potentialdifferenz aufweisen, dass Zellen resp. Vesikel elektrophoretisch auf die Apertur zubewegt werden. Die Zugabeöffnung kann von beliebiger Form sein, üblicherweise ist sie aber ellipsenförmig, insbesondere kreisförmig, so dass sie sich z.B. konzentrisch über der Apertur anordnen lässt.

Die Fixierung des Trägers zwischen den Elektroden kann dadurch erfolgen, dass zwischen der jeweiligen Elektrode und dem Träger je ein Abstandhalter vorgesehen wird, der, ebenso wie der Träger selbst, aus elektrisch isolierendem Material besteht und Kanäle aufweist, die zwischen der Apertur und der Elektrode angeordnet sind und mit diesen in Kontakt stehen. Mit leitfähiger Lösung gefüllt können diese Kanäle als Referenz resp. Probekammer dienen. Dabei hat es sich als günstig erwiesen, wenn die Referenzkammer so geringe Ausmasse aufweist, dass die darin enthaltene Referenzpufferlösung durch Kapillarkräfte darin fixiert ist. Im Extremfall ist es möglich, das Referenzvolumen ohne physische Begrenzungen und nur über Kapillarkräfte zwischen Chip und Elektrode zu fixieren. Das Probenkompartiment (die Probenkammer) bildet sich zwischen der Chipoberfläche und der Zugabe-Elektrode aus. Es hat keine seitliche Begrenzung sondern wird durch Kapillarkräfte gehalten. Im Sinne einer Integration dieser Methode ist es aber auch möglich eine Probenkammer mit seitlichen Begrenzungen aufzubauen. Die Messanordnung der vorliegenden Erfindung umfasst Ausführungsformen mit Probenkammern sowohl ohne als auch mit physischen seitlichen Begrenzungen.

Da es je nach angestrebter Analyse sinnvoll ist, die Membran beidseitig mit Messlösung in Kontakt zu bringen, kann eine Zugabe eines zu untersuchenden Stoffes selbstverständlich auf von der üblicherweise als Referenzseite dienenden Seite her erfolgen. Auch kann z.B. ein Referenzpuffer in ein pastöses Gel eingebracht werden, wodurch ein Austausch der ausserhalb des Gels liegenden Flüssigkeit möglich wird, ohne den im Gel gespeicherten Referenzpuffer in seiner Zusammensetzung zu verändern. Als solche Gele eignen sich beispielsweise Agarose und Polyacrylamid.

Das erfindungsgemässe Messverfahren erlaubt insbesondere die Messung von Ionenkanalströmen auf zuverlässige und reproduzierbare Weise und dies mit einem hohen Signal-Rauschabstand. Grundlage dafür ist die genaue Positionierung und anschliessende elektrisch dichte Bindung von Vesikeln, Zellen oder anderen biologischen Organellen, resp. Membranen entsprechenden Ursprungs, an mikrostrukturierten Öffnungen (im folgenden auch als Apertur bezeichnet) mit einem Durchmesser $dM < 15\ \mu m$, vorzugsweise $< 10\ \mu m$, insbesondere 0,3 - 7 um, speziell bevorzugt 0,3 - 5 $\mu m$ und ganz speziell bevorzugt 1 - 5 $\mu m$. Die elektrisch dichte Bindung der Vesikel bzw. Zellen resp. deren Membranen wird durch eine starke elektrostatische Anziehung zwischen der Trägeroberfläche und der Membranoberfläche erreicht.

Für das erfindungsgemässe Verfahren hat es sich als günstig erwiesen, wenn die Membran auf einen möglichst planaren Träger aufgebracht wird. Ein entsprechender Träger kann aus diversen Materialien bestehen; Vorteilhaft für geeignete Materialien ist aber, dass sie vorzugsweise nicht nur mikroskopisch flach sondern molekular relativ eben sind. Zudem müssen geeignete Materialien im System inert, nicht leitend und vorzugsweise chemisch modifizierbar sein.

Als speziell geeignet herausgestellt haben sich mikrostrukturierte Silizium/Siliziumoxyd bzw. Silizium/Siliziumoxynitrid Träger, die, für die Bereitstellung einer guten elektrostatischen Anziehung, mit einer die gewünschte Oberflächenladung verleihenden Substanz beschichtet sind. Geeignet sind beispielsweise Polykationen, wie von Mazia, Schatten et al. beschrieben (siehe D. Mazia, G. Schatten et al. (1975). "Adhesion of cells to surfaces coated with polylysine." J. Cell. biol. 66: 198-200). Solche Polykationen sind beispielsweise Poly-L-Lysin und Polyethylenimin.

Bei der Auswahl geeigneter Trägerchipmaterialien selbst ist, wie bereits oben erwähnt, auf eine ausreichende Modifizierbarkeit der Oberfläche zu achten, damit eine elektrostatische oder gegebenenfalls van der Waals oder kovalente Bindung von Vesikeln oder biologischen Zellen resp. entsprechender Membranen oder Membranfragmente, daran möglich ist. Zudem ist eine Bindung aufgrund hydrophob- hydrophiler Wechselwirkungen unter bestimmten Umständen möglich (Radler, J., H. Strey, et al. (1995). "Phenomenology and Kinetics Of Lipid Bilayer Spreading On Hydrophilic Surfaces." Langmuir 11(11): 4539-4548). Ferner muss das Trägermaterial bearbeitbar, d.h. mit einer Apertur resp. einem Fenster gewünschter Grösse versehbar sein und eine Fokussierung des elektrischen Feldes auf die Apertur ermöglichen.

Ein speziell geeigneter Träger ist ein $Si/SiO_2$-bzw. Silizium/Siliziumoxynitrid-Chip, der aus handelsüblichen Si-Wavern mit einer Oxidschicht einer Dicke D von üblicherweise > 200 nm herstellbar ist. Ein solcher Träger lässt sich leicht mikrostrukturieren. Nach beispielsweise Fotolithographie, bzw. bei Aperturen von $d < 1,5 \ \mu m$ Elektronenstrahllithographie, können die Strukturen durch anisotropes Ätzen des Siliziums in KOH-haltigem Medium sowie reaktives Ionenätzen der Quarzschicht gewonnen werden. Neben Quarz bilden auch Glasschichten, feste oder gelartige Polymere etc. geeignete modifizierbare Oberflächen aus. Weiterhin eignen sich beispielsweise Plastomere und Elastomere wie Polyimide, Polymethylmetacrylate, Polycarbonate, Silicagele wie Sylgard, etc.

Wesentlich an solchen Strukturen ist die Grösse der Apertur, welche < 15 $\mu m$ betragen sollte, meist < 10 $\mu m$, insbesonders < 7 $\mu m$ und bevorzugt < 5 $\mu m$, sowie die Grösse des Fensters in der Oberflächenschicht, z.B. dem Quarz, welches vorzugsweise < 50 $\mu m$ ist, im Idealfall aber auf die Grösse der Apertur reduziert ist, um unter bestimmten Umständen (geringe Pufferleitfähigkeit) eine starke Fokussierung des elektrischen Feldes auf die Apertur zu unterstützen, aber vor allem um mechanische Spannungen (Bruchgefahr)zu reduzieren. Eine starke elektrische Fokussierung entsprechend der starken Inhomogenität des **E** - Feldes (Betrag **E** nimmt mit Annäherung an die Apertur zu) erlaubt mit $F_{elektrisch} \sim E$ (F = Kraftvektor, E = elektrisches Feld) eine entsprechend genaue Bewegung der Vesikel auf die Apparatur. Selbstverständlich kann ein Träger viele Aperturen enthalten, die sequentiell oder parallel für Messungen genutzt werden.

Ein planarer, mit mindestens einer Apertur versehener Trägerchip wird zwischen zwei Elektroden gegeben. Geeignete Elektroden sind z.B. Ag/AgCl, Pt; infolge ihrer leichten Herstellbarkeit werden aber Ag/AgCl-Elektroden bevorzugt. Die Elektroden dienen neben der Spannungsklammerung (voltage clamp) insbesondere auch der Positionierung der Vesikel und Zellen resp. entsprechender Membranen. Die Elektroden befinden sich üblicherweise im Abstand von 0,5 bis 3 mm, meist 0,5 - 1 mm vom Träger, können aber auch weiter entfernt sein. Eine symmetrische Anordnung ist bevorzugt aber nicht notwendig.

Durch einen planaren und in der Vertikalen leicht realisierbaren optisch transparenten Aufbau, z.B. bei Verwendung planarer punktierter Elektroden, bzw. ausserhalb der durch die Apertur hindurch gehenden Vertikalen angeordneter Punktelektroden, eignet sich das oben beschriebene System für simultane elektrische und optische (Fluoreszenz) Messungen. Durch neue Fluoreszenztechniken, wie die Fluoreszenz-Korrelationsspektroskopie und die konfokale CCD-Beobachtung, ist die optische Detektion von Liganden-Bindungen an einzelne Rezeptoren möglich geworden. Die Kombination solcher optischer Techniken mit der hier vorgestellten Methode erlaubt erstmalig die Unterscheidung bzw. Auflösung von Liganden-Bindungsereignissen und Kanalaktivitäten. Damit können beispielsweise wichtige Informationen über die Stabilisierung von Konformationsänderungen des Rezeptors durch die Liganden-Bindung und über die funktionelle Verschiedenheit der Liganden-Bindungsplätze im Rezeptor gewonnen werden. (J. Edelstein, O. Schaad, J.-P. Changeux (1997). "Single Binding versus Single Channel Recordings: A new Approach to Study Ionotropic Receptors", Biochemistry 36: 13755-13760). Solche Ergebnisse sind wichtig für das Verständnis der Wirkungsweise von Agonisten und Antagonisten und damit für die Entwicklung neuer Medikamente.

Die vielfältige Anwendbarkeit der durch die Erfindung ermöglichten Messvorrichtung lässt sich durch Multiarray-Auslegung noch verbessern. Durch Mikrostrukturierung lassen sich auf kleinstem Raum diverse Aperturen anbringen, die entweder an die gleichen Elektroden gekoppelt sind, oder aber getrennte Messkompartimente darstellen, da z.B. Ag/AgCl-Elektroden ebenfalls leicht mikrostrukturierbar sind.

Ferner ist es möglich die durch die Erfindung ermöglichte Messanordnung mit Vorrichtungen für die Probenzugabe und den Probenaustausch, für die Probenauftrennung und die Regulierung der Messung zu koppeln, z.B. durch Verbindung der Kompartimente über Schläuche mit einem Pumpsystem oder einer Vorrichtung die mittels hydrostatischer Druckdifferenzen oder Piezotropfenverfahren resp. Tintenstrahlverfahren oder Kontakttransferverfahren oder elektroosmotischen Verfahren oder temperaturkontrollierten Verfahren oder Kapillarelektrophorese (CE) oder HPLC (High Pressure Liquid Chromatography) funktioniert.

Die Messung kann durch Zugabe von membranaktiven Stoffen beeinflusst werden, z.B. durch Zugabe von Porenbildnern, Proteoliposomen und Membranproteinen.

Die Erfindung wird in der Folge anhand der Figuren näher erläutert.

Kurze Beschreibung der Zeichnungen

Figur 1 ist eine schematische, nicht massstabgetreue und nicht detailgetreue Darstellung eines aus Si/SiO$_2$ hergestellten Trägerchips.

Figur 2 ist eine elektronenmikroskopische Aufnahme einer in die SiO$_2$-Schicht geätzten Apertur in verschiedenen Ansichten: (A) Apertur von der SiO$_2$ Oberflächenseite aufgenommen (B) Apertur von der Si - Seite aufgenommen (C) Überblick SiO$_2$-Seite (D) Überblick anisotrop geätzte Si - Seite

Figur 3 ist eine schematische, nicht massstabgetreue und nicht detailgetreue Darstellung des Messaufbaus mit planparallelen Elektroden im Schnitt.

Figur 4 ist eine schematische, nicht massstabgetreue und nicht detailgetreue Darstellung des Messaufbaus mit Punkt- bzw. Draht-Elektroden im Schnitt.

Figur 5 zeigt Rhodamin-markierte Vesikel (Membran in Natura rot, auf Bild hellgrau) nach 24 Stunden Reinigung, bei welcher die Anzahl kleiner Vesikel (d < 5 $\mu$m) gegenüber der ungereinigten Lösung stark reduziert wurde. Die Vesikel enthalten 200 mM Sorbitollösung, die mit Carboxyfluorescein (in Natura grün, auf Bild mittelgrau) fluoreszenzmarkiert ist.

Figur 6 zeigt Vesikel nach der Bindung an Poly-L-Lysin beschichtete Oberflächen, ausgezogen zu sehr flachen Gebilden, die keine Carboxyfluorescein Fluoreszenz aufweisen.

Figur 7 zeigt einen Querschnitt durch fusionierte Vesikel, mit einem Kalibrierbalken von 5 $\mu$m Länge.

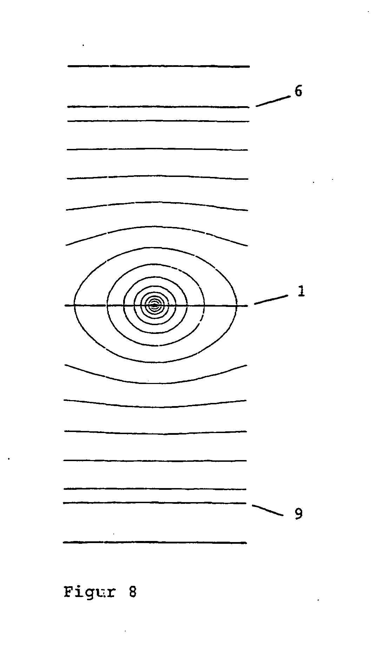

Figur 8 zeigt eine Finite Elemente Simulation (FEM) der elektrischen Feldverteilung um einen Chip mit 4 um Apertur zwischen parallelen Elektroden. Als Parameter wurden verwendet: C$_{Puffer}$ = 10 mM KCl, d$_{Apertur}$ = 4 $\mu$m und Abstand Chipapertur(4) - Elektrode(6,9) = 1 mm. Die Äquipotentiallinien haben eine Abstand von 4 mV, wobei die Potentialdifferenz zwischen den Elektroden 80 mV beträgt. Der Feldlinienverlauf ist in dieser Simulation durch die Annahme von Leckströmen im Randbereich des Trägerchips ellipsenförmig verzerrt (normal: kreisrund).

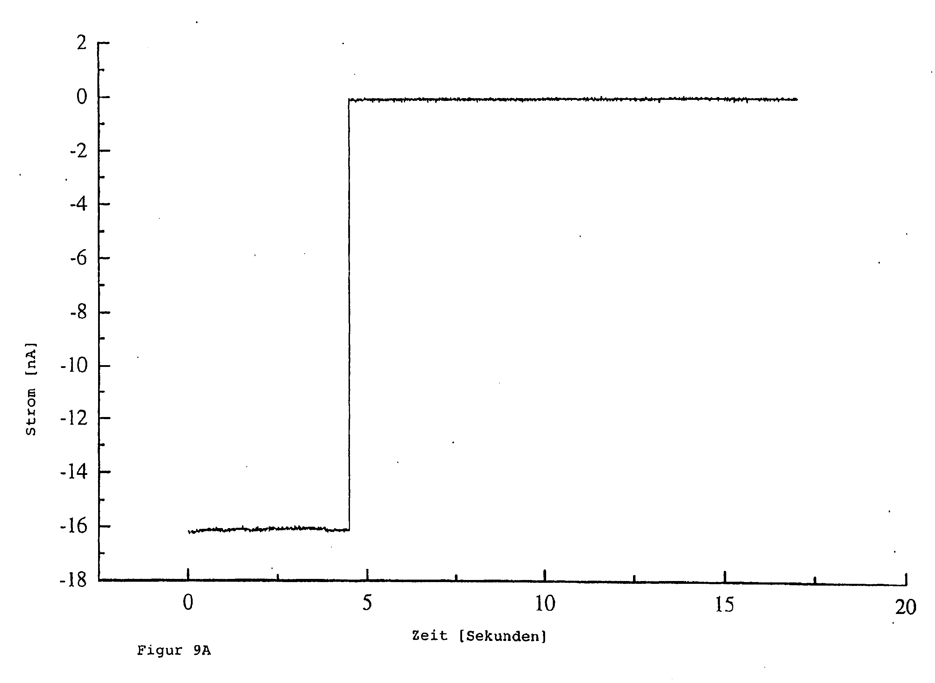

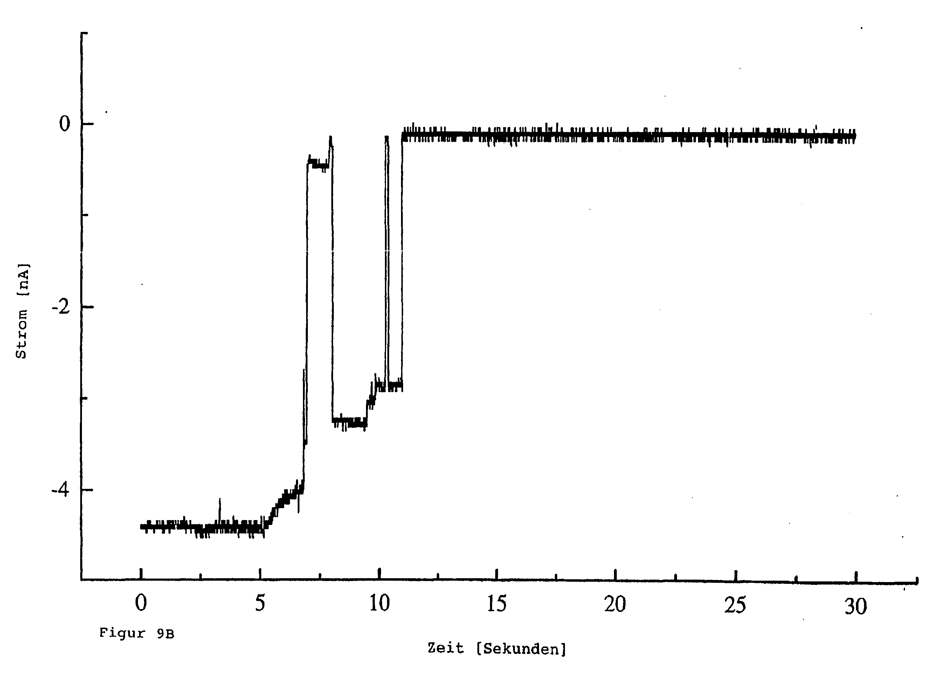

Figur 9 zeigt den zeitlichen Verlauf der Vesikelbindung und der Ausbildung einer Membran mit sehr hohem elektrischem Abdichtwiderstand bei einer Apertur von 4 $\mu$m (Figur 9A) und 7 $\mu$m (Figur 9B), sowie 10 mM KCl, einer Klemmspannung von -80 mV und PLL beschichteter SiO$_2$ Oberfläche (PLL = Poly-L-Lysinbromid).

Figur 10 zeigt den Durchgang von Vesikeln durch eine Apertur von 7 $\mu$m als Modulation im Strom-Zeit-Diagramm bei einer Klemmspannung V$_C$ = -80 mV konstant.

Figur 11 zeigt im Strom-Zeit-Diagramm dass die Zugabe von Ca$^{2+}$ mit einer Endkonzentration von 4 mM nach dem Andocken von Vesikeln an die unmodifizierte Apertur (7 $\mu$m) zu einem elektrisch hochdichten Abschluss zwischen Chipoberfläche und Vesikelmembran führt.

Figur 12 zeigt das zeit- und spannungsabhängige Schalten von Alamethicinporen in einer auf dem Chip erzeugten Membran (C$_{Alamethicin}$ = 0.1 $\mu$g/ml in 85 mM KCl) bei negativen Potentialen.

Figur 13 gibt Änderungen des Membranwiderstandes einer auf einem Si/SiO$_2$ Trägerchip erzeugten Membran nach der Fusion mit nAChR (nicotinischer Acetylcholin Receptor) enthaltenden Vesikeln wieder.

(A) Zufällige Rezeptoröffnungen in Abwesenheit von Liganden bei 400 mM KCl und positiven Potentialen. (B) 150 Sekunden nach Zugabe des nAChR Agonisten Carbamylcholin (20 $\mu$M Endkonzentration) sind keine Rezeptoröffnungen mehr zu beobachten (Desensitisierung).

Wege zur Ausführung der Erfindung

Das Verfahren resp. die durch die Erfindung ermöglichte Vorrichtung (Messanordnung) der vorliegenden Erfindung sind speziell geeignet für den Einsatz beim Wirkstoffscreening, als Ersatz konventioneller Patch-clamp-Techniken und als portable Biosensoren, beispielsweise für die Umweltanalytik. Beispielhafte Messanordnungen sowie Anwendungsgebiete derselben werden in der Folge etwas näher beschrieben.

Die durch die Erfindung ermöglichte Messanordnung weist mindestens zwei Elektroden 6, 9 und separierte, für die Aufnahme von Flüssigkeit geeignete Kompartimente auf und ist dadurch gekennzeichnet, dass sich zwischen zwei gegenüber befindlichen, in je mindestens ein Kompartiment hineinreichenden oder ein solches berührenden, und beliebig geformten Redoxelektroden 6, 9 ein Träger 1, befindet, der mindestens eine Apertur 3 enthält und jeweils mindestens 2 Kompartimente voneinander trennt.

Vorzugsweise weist sie auf einer Seite oder auf beiden Seiten des Trägers 1 Mittel auf, die eine Flüssigkeitszugabe, eine Flüssigkeitsspeicherung, und gegebenenfalls einen Flüssigkeitsaustausch, sowie die Zugabe von Zellen, Vesikeln, anderen biologischen Organellen oder Teilen derselben zwischen Träger und Elektrode ermöglichen. Die Apertur 3 weist einen solchen Durchmesser auf, dass sich bei Existenz einer Spannungsdifferenz über dem Chip, vermittelt durch die Elektroden 6, 9, ein inhomogenes elektrisches Feld um die Apertur aufbaut, das mit Annäherung an die Apertur betragsmässig grösser wird und nahe der Apertur Vesikel, Zellen, Zellfragmente oder biologische Organellen elektrophoretisch auf diese zubewegen kann. Des weiteren bevorzugt ist, dass der Träger 1 eine elektrisch geladene Oberfläche 5 aufweist, die attraktiv für biologische Membranen ist, oder eine Oberfläche 5 aufweist, die eine molekülspezifische oder durch multivalente Ionen vermittelte Bindung von Zellen, Vesikeln, Membranfragmenten oder biologischen Organellen daran gestattet. Ein solcher Träger ist beispielsweise ein Silicium-Trägerchip mit aufgebrachter Oxyd- oder Oxynitridschicht. Eine elektrisch geladene Oberfläche 5 kann auch durch Modifikation, insbesondere mittels Polykationen und/oder Silanen, z.B. Aminosilanen, erzeugt werden, oder der Träger kann eine Beschichtung 2 mit elektrisch geladener Oberfläche 5 aufweisen. Der Träger 1 kann zusätzlich vor der Modifikation seiner Oberfläche oder vor seiner unmittelbaren Benutzung in einem Sauerstoffplasma gereinigt und teilweise oder vollständig hydrophylisiert werden.

Aufgrund der speziellen Anordnung ist es nicht notwendig, dass die Messanordnung Kompartimente mit physischer Begrenzung aufweist.

In einer bevorzugten Ausführungsform der Messanordnung sind jeweils eine Elektrode und mindestens eine Apertur 3 im Träger 1 über einen Kanal oder eine Kammer 8 in einem Abstandhalter 7, 10 unter Ausbildung eines offenen oder geschlossenen Kompartiments miteinander verbunden.

Auch können mehr als zwei Elektroden 6, 9 und mehr als eine Apertur 3 vorhanden sein, derart, dass mindestens eine Elektrode, z.B. eine Referenzelektrode, der Messung über mehr als eine Apertur 3 dient, oder die Messanordnung kann einen Träger 1 mit mehr als einer Apertur 3 und doppelt so vielen Elektroden 6, 9 wie Aperturen 3 aufweisen, derart, dass sich jeweils eine Apertur 3 zwischen jeweils zwei Elektroden 6, 9 befindet.

Ferner können die Kompartimente über Schläuche mit einem Pumpensystem oder einem Gerät, das auf hydrostatischer Druckbasis oder mittels eines Piezotropfenverfahrens resp. Tintenstrahlverfahrens oder mittels eines Kontakttransferverfahrens oder elektroosmotischen Verfahrens oder temperaturkontrollierten Verfahrens arbeitet, gekoppelt sein, derart, dass Flüssigkeiten resp. Proben in beliebige Kompartimente zugegeben oder darin ausgetauscht werden können.

Auch kann die Messanordnung mit einer Apparatur zur Erzielung einer Probenauftrennung, insbesondere Kapillarelektrophorese (CE) und HPLC, gekoppelt sein und der Analyse der aufgetrennten Substanzen dienen, oder sie kann mit Mitteln versehen sein, die der kontinuierlichen oder regelmässigen Prüfung des Flüssigkeitsstands in den Kompartimenten dienen, sowie mit Mitteln für die Nachregelung entsprechend voreingestellter Füllparameter.

In einer weiteren Ausführungsform kann die Oberfläche 5 des Trägers 1 derart strukturiert sein, dass sich hydrophile und hydrophobe Bereiche ergeben, wobei sich die hydrophilen Bereiche vorzugsweise um die Apertur befinden.

Solche Messanordnungen lassen sich z.B. für die nachfolgend näher beschriebenen Messungen verwenden:

Wirkstoffscreening:

Die vorliegende Erfindung eignet sich ausgezeichnet für die Sondierung einer grossen Anzahl von potentiellen Liganden, die mittels der kombinatorischen Chemie in jeweils kleinen Mengen herstellbar sind. Andererseits sind auch viele Rezeptorproteine, vor allem ligandengesteuerte und G-Protein-gekoppelte Rezeptoren, nur in sehr begrenzten Mengen verfügbar. Dank dem erfindungsgemässen Verfahren resp. der erfindungsgemässen Messanordnung/Messvorrichtung ist es möglich, mit sehr wenigen Zellen zu arbeiten, entweder direkt oder nach vorheriger Isolation und Rekonstitution der Rezeptorproteine in Vesikel bzw. Lipidmembranen. Durch das unkomplizierte Anordnen der Sensorelemente in Arrays können verschiedene Substanzen oder Rezeptoren zeitlich simultan selektiert werden. Es besteht zudem die Möglichkeit, die Rezeptoraufreinigung und Rekonstitution in Lipidvesikel mikrochromatographisch in in die erfindungsgemässe Vorrichtung wahlweise integrierbaren on-chip Containern vorzunehmen.

Ersatz konventioneller Patch-clamp-Techniken:

Wie bereits eingangs erwähnt bilden konventionelle Patch-clamp-Techniken die Grundlage für die Untersuchung der Funktionalität von Membranrezeptoren sowie allgemein der Veränderung membranären Eigenschaften als Antwort auf signal- und metabolische Prozesse in Zellen. Dienen als Untersuchungsobjekt isolierte Zellen einer homogenen Zellpopulation, wie dies beispielsweise bei transformierten Zellen oft der Fall ist, kann das erfindungsgemässe Verfahren als mindestens gleichwertiger Ersatz zur Patch-clamp-Technik dienen. Als Untersuchungsobjekte für dieses Verfahren eignen sich z.B. dissoziierte Neuronen und kultivierte Säugerzellinien sowie Pflanzenprotoplasten.

Portable Biosensoren/Umweltanalytik:

Die ausgezeichnete mechanische Stabilität des durch die Erfindung ermöglichten Messsystems bzw. dessen praktisch automatischer Membranaufbau erlauben dessen Verwendung in Biosensoren. Durch Verwendung geeigneter transformierter Zellen oder in Vesikeln rekonstituierter Rezeptoren oder kanalbildender Proteine können Sensoren aufgebaut werden, die für sehr verschiedene Substrate bzw. Metaboliten sensitiv sind. Dabei ist bei sehr guter Membranabdichtung (seal formation), wie sie dank der erfindungsgemässen Vorrichtung erzielt wird, die Empfindlichkeit prinzipiell nur von der Bindungskonstanten des Rezeptors abhängig und kann z.B. bei G-proteingekoppelten Rezeptoren unter einem Nanomol und bei ionotropen Rezeptoren (z.B. 5 HT3, nAChR, GABA$_A$R, GlycineR, GluR) im nanomolaren Bereich liegen (North, R. A. (1994). Ligand- and voltage-gated ion channels, CRC Press; Peroutka, S. J. (1991). Serotonin receptor subtypes - Basic and clinical aspects. New York, John Wiley & Sons; Peroutka, S. J. (1994). G Protein coupled receptors, CRC Press; Conley, E. C. (1996). The ion channel facts book, Academic Press).

Dem erfindungsgemässen Messverfahren liegt das folgende bekannte Messprinzip zugrunde:

Die elektrischen Eigenschaften transmembranären Ionenkanäle oder ionotroper Rezeptoren werden im allgemeinen mit sogenanntem voltage-clamp-Techniken (z.B. klassisches Voltage-Clamp, Patch-Clamp und Oocyten-Voltage-Clamp) charakterisiert (siehe Hamill, Marty et al. 1981 a.a.o; J.G. Nicholls, A.R. Martin et al. (1992). From neuron to brain: a cellular and molecular approach to the function of the nervous system. Sunderland, Ma., Sinauer Associates, Inc.). Dazu wird über der Membran, die den oder die betreffenden Ionenkanäle enthält, eine elektrische Potentialdifferenz angelegt und gleichzeitig der zur Aufrechterhaltung dieser Differenz notwendige Strom analysiert. Entsprechend dem Ohmschen Gesetz, wonach V = I x R bzw. I = V / R ist, liefert diese Analyse eine Aussage über die Leitfähigkeit und damit - allerdings nicht eindeutig - verbunden eine Aussage über den Konformationszustand des kanalbildenden Proteins. Daraus lassen sich Ligandenbindungsereignisse, Spannungsabhängigkeiten usw. ermitteln.

Da der Ionenfluss durch ionotrope Membranproteine mit 0.1 - 50 pA bei $V_M$ = -60 mV Membranspannung im allgemeinen sehr klein ist, muss für ein akzeptables Signal-Rauschverhältnis die Varianz der auftretenden Leckströme etwa um den Faktor 5-10 unter den zu messenden Signalen liegen. Diese Leckströme treten im wesentlichen zwischen der Membran und deren Befestigung auf und stellen das prinzipielle Hauptproblem bei allen Voltage Clamp Techniken dar.

Das Problem kann verschiedenartig gelöst werden, z.B. durch Vergrösserung des zu vermessenden Membranareals und damit, durch Aufsummation, eine Vergrösserung der Ionenströme. Allerdings geht dabei, gerade in biologischen Systemen, die Spezifität verloren. Es wird dann im allgemeinen keine eindeutige oder vollkommen artefaktfreie Aussage, z.B. bei Ligandenzugabe, mehr möglich sein.

Das Problem des ausreichenden Signal-Rauschabstandes kann aber auch gelöst werden, indem ein sehr hoher Abdichtwiderstand (seal) zwischen Membran und Elektrode aufgebaut wird. Dieses Prinzip wird bei der vorliegenden Erfindung angewendet. Dazu wird ein planarer Trägerchip mit einer für Zellen und Vesikel stark adhäsiven Oberfläche verwendet. Dieser Chip trennt die bei der Messung auf unterschiedliche Potentiale geklemmten zwei Kompartimente, wobei sich in dessen Mitte eine (sub)mikrometergrosse Öffnung befindet. Diese Öffnung oder Pore (Apertur) ist mit Referenzpufferlösung gefüllt und wird während der Messung durch die starke Bindung von Zellen bzw. Vesikeln an die Oberfläche elektrisch dicht verschlossen. Diese elektrisch sehr dichte Bindung erlaubt die Messung auch sehr kleiner (0.1 pA) Ionenströme.

Ähnliche Ansätze mit Lipidmembranen, die jedoch ohne Träger mit den erfindungsgemässen Anforderungen resp. der erfindungsgemässen Modifizierung der Oberfläche auskamen, waren bisher, bedingt durch das Auftreten zu geringer Abdichtwiderstände der Membran, für sensitive Messungen nicht einsetzbar. Dazu gehören LB Transfers (R.Coronado und R. Latorre (1983). "Phospholipid bilayers made from monolayers on patch-clamp pipettes." Biophys. J. 43(2): 231-6; D.P. Nikolelis und C.G. Siontorou (1995). "Bilayer lipid membranes for flow injection monitoring of acetylcholine, urea, and penicillin." Anal. Chem. 67(5): 936-44; T.D. Osborn und P. Yager (1995). "Formation of planar solvent-free phospholipid bilayers by langmuir-blodgett transfer of monolayers to micromachined apertures in silicon." Langmuir 11(1): 8-12; T.D. Osborn und P. Yager (1995). "Modeling success and failure of Langmuir-Blodgett transfer of phospholipid bilayers to silicon dioxide." Biophys. J. 68(4): 1364-73 und Vesikel Spreitung (P. Nollert, H. Kiefer et al. (1995). "Lipid vesicle adsorption versus formation of planar bilayers on solid surfaces." Biophys. J. 69(4): 1447-55; J. Radler, H. Strey et al. (1995). "Phenomenology and Kinetics Of Lipid Bilayer Spreading On Hydrophilic Surfaces." Langmuir 11(11): 4539-4548). Ein miniaturisierter Black Lipid Membrane (BLM) Aufbau wurde von Eray, Dogan et al. 1995 berichtet (siehe M. Eray, N.S. Dogan et al. (1995). "A highly stable and selective biosensor using modified nicotinic acetylcholine receptor (nAChR)." Biosystems 35(2-3): 183-8).

Kritischer Punkt beim erfindungsgemässen Verfahren ist die genaue Positionierung der Zellen und Vesikel über der Pore (Apertur). Diese Positionierung wird durch die erfindungsgemässe Elektrodenanordnung und Feldfokussierung erzielt. Der Abstand zwischen Mess- und Referenzelektrode beträgt üblicherweise < 6 mm, kann aber auch grösser sein. Dabei befinden sich die Mess- und Referenzelektrode jeweils im Abstand von ca. 0,2 bis 3 mm, vorzugsweise 0,5-2 mm, insbesondere 0,5 bis 1 mm unter- und oberhalb des Trägerchips. Verwendet wird eine Klemmspannung, die für eine elektrophoretische Positionierung der Vesikel auf die Apertur ein wirksames elektrisches Feld erzeugt. Diese Spannung ist nicht kritisch, sie liegt aber üblicherweise im Bereich $V_c$ = -30 bis -300 mV, insbesondere -60 bis -100 mV und speziell bevorzugt -60 bis -80 mV. Durch die damit bedingte elektrophoretisch treibende Kraft werden Vesikel und Zellen, dem elektrischen Feld folgend, genau auf die Chipöffnung zubewegt. Da das Feld **E** stark inhomogen ist und sich mit Annäherung an die Apertur betragsmässig stark vergrössert, werden Vesikel automatisch auf die Apertur zubewegt. Da die elektrophoretisch wirksamen Feldstärken vor allem nahe der Apertur wirksam werden (Distanz zur Apertur < 200 $\mu$m), müssen die Zellen/Vesikel in diesen Bereich hineingebracht werden oder konvektiv dorthin gelangen. Dazu kann sich in der Messelektrode ein Loch (z.B. d < 1 mm) gegenüber der Trägerchipöffnung (Apertur) befinden.

Für alle Messungen ist es wichtig, dass der Öffnung- resp. Aperturdurchmesser wesentlich kleiner als der Durchmesser der Vesikel oder biologischen Zellen ($d_{Zelle}$, $d_{Vesikel}$ » $d_{Apertur}$) ist. Für die Aperturen von d > 2 $\mu$m werden deshalb vorzugsweise Vesikel mit Durchmessern d > 20 $\mu$m verwendet.

Die charakteristischen elektrischen Eigenschaften lassen sich wie folgt mathematisch beschreiben:

Das thermische Rauschen $\sigma$ einer zirkulären Lipidmembran ist prop. zu $R_M$-1/2 (B. Sakmann und E. Neher (1983). Single-channel recording. New York, London, Plenum Press.):

$$\sigma = \sqrt{\frac{4kTf_c}{R_M}},$$

mit $R_M = A_{spec}/(\pi r_M^2)$ folgt daraus:

$$\sigma = r_M \sqrt{\frac{4\pi kTf_c}{R_{spec}}}$$

In diesen Formeln bedeutet $\sigma$ den effektiven Rauschstrom, r den Radius, f die Frequenz, k die Bolzmann Konstante, R den Widerstand und T die Temperatur.

Damit ergibt sich als Konsequenz für eine zu Messzwecken erfolgreich einsetzbare Membran, dass $r_M/\sqrt{R_{spec}}$ sehr klein sein muss. Die Minimierung dieses Produktes kann erfindungsgemäss auf 2 Wegen beschritten werden, einerseits durch Minimierung des Membranradius rM und andererseits resp. zusätzlich durch den elektrisch dichten Abschluss der verwendeten Membranen.

Die mechanische Stabilität der Membran ist abhängig von deren Grösse. Die Grösse der Apertur in den Trägern bedingt den Durchmesser der zu bildenden Membran. Vorzugsweise besitzen die Apertur und das Fenster einen vergleichbaren Durchmesser. Da die zur Auslenkung einer Membran notwendige Kraft proportional zu $r_M^{-2}$ ist, folgt bei Strukturen von $d_{Apertur}$ < 5 $\mu$m und folglich dM < 5 $\mu$m eine extreme Zunahme der Membranstabilität verglichen mit typischen Öffnungsweiten von $d_{Apertur}$ > 100 $\mu$m bei konventionellen BLM Systemen.

Wie bereits früher ausgeführt können Träger für Lipidmembranen aus verschiedenen Materialien hergestellt werden, aufgrund ihrer guten und exakten Bearbeitbarkeit werden aber Si/SiO$_2$ und Silizium/Siliziumoxynitrid Träger bevorzugt.

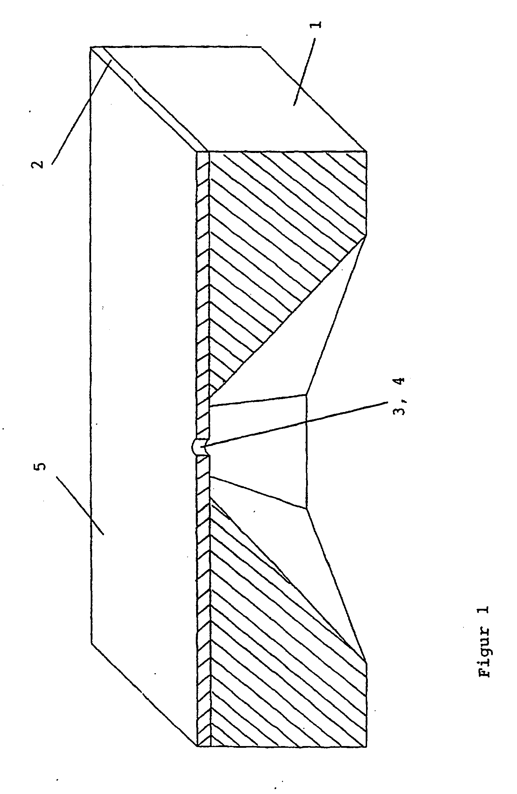

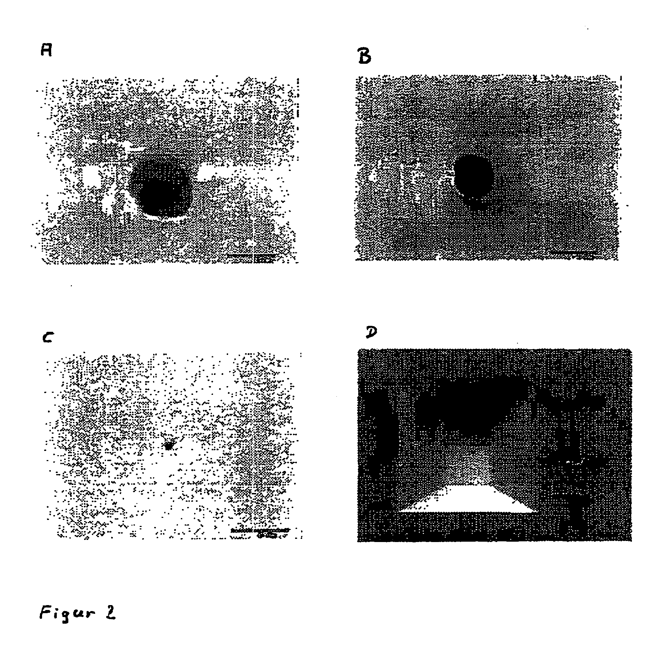

Die als Träger (Fig. 1) für die Lipidmembranen bevorzugt verwendeten Si/SiO$_2$ Chips können aus handelsüblichen Si-Wavern 1 mit einer Oxyd- oder Oxynitridschichtdicke 2 von gewöhnlich > 200 nm hergestellt werden. Nach Fotolithographie bzw. bei Aperturen 3 von d < 1.5 $\mu$m Elektronenstrahllithographie werden die Strukturen durch anisotropes Ätzen des Siliziums in KOH-haltigem Medium sowie reaktives Ionenätzen der Quarzschicht gewonnen. Ein entsprechender Träger ist in Figur 1 schematisch dargestellt, eine fotographische Aufnahme der Apertur zeigt Figur 2.

Wesentlich an diesen Strukturen ist die Grösse der Apertur, die wesentlich kleiner als die verwendeten Vesikel, Zellen bzw. Organellen sein sollte. Eine Verringerung der Aperturgrösse ist für die Ausbildung eines hochwertigen Seals von Vorteil, führt aber anderseits zu einer Verringerung des elektrischen Attraktionsbereiches um die Apertur. Aperturen von 0.3 bis 7 $\mu$m ergeben eine ausgezeichnete Sealwahrscheinlichkeit und -qualität. Die Grösse des Fensters 4 (Fig. 1) ist im Idealfall auf die Grösse der Apertur 3 reduziert.

Bei der Auswahl geeigneter Trägerchipmaterialien ist neben der Bearbeitbarkeit ausserdem auf eine ausreichende Modifizierbarkeit der Oberfläche zu achten, damit eine elektrostatische oder gegebenen kovalente Bindung von Vesikeln oder biologischen Zellen daran möglich ist. Es hat sich hier gezeigt, dass eine mehrminütige Behandlung der Trägerchips in O$_2$ - Plasma vor der eigentlichen Oberflächenmodifizierung sehr zu konsistenten Oberfächeneigenschaften beiträgt.

Um eine starke Adhäsion der Vesikel zu gewährleisten, wird die. Oberfläche des Trägers gegebenenfalls mit einem Haftverbesserer, beispielsweise Polykationen, beschichtet (siehe Mazia, Schatten et al., a.a.O. 1975). Für die Physisorption kann beispielsweise eine wässrige Lösung von Polykationen, z.B. 0.1 % Poly-L-Lysinbromid (Sigma), MW 100 000, direkt vor der Messung für 2-5 Min. auf den Träger gegeben und anschliessend mit Messpufferlösung abgespült werden. Die kovalente Bindung peptidischer Polykationen erfolgt vorzugsweise über vorher aktivierte Hydroxylgruppen der Quarzoberfläche, z.B. mittels Tosylchlorid (Triphenylchlormethan) (M.L. Williamson, D.H. Atha et al. (1989). "Anti-T2 monoclonal antibody immobilization on quartz fibers: stability and recognition of T2 mycotoxin." Analytical Letters 22(4): 803-816).

Durch die Modifizierung der Trägeroberfläche erreicht man eine Anziehung von Vesikeln mit negativer Oberflächenladung, die vollkommen ausreichend für elektrisch hochdichte Abschlüsse zwischen Membran und Trägeroberfläche ist.

Die Vesikel- resp. Zellbindung kann auch durch molekülspezifische Wechselwirkungen, z.B. Biotin-Streptavidin oder Histidin-NTA (Histidin-Nitrilotriacetic acid) vermittelt werden.

Alternativ zu der beschriebenen Verwendung von Polykationen kann die Oberfläche auch durch andere Verbindungen mit Kationeneigenschaften im gewünschten pH-Bereich, wie z.B. 4-Aminobutyl-dimethyl-methoxysilan, modifiziert werden.

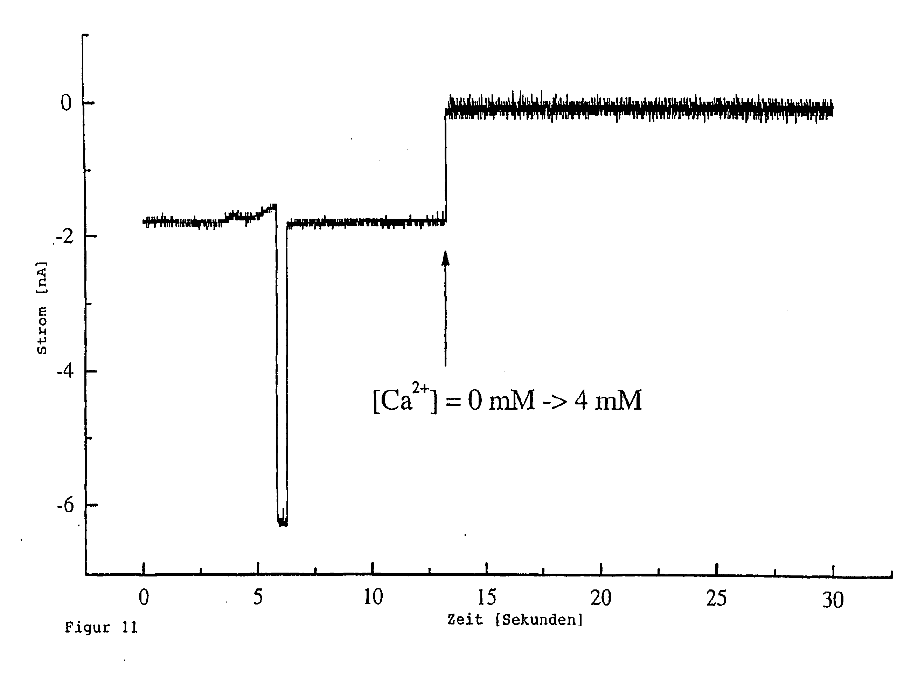

Eine weitere Möglichkeit für die elektrisch dichte Bindung von Vesikeln an die $SiO_2$-Oberfläche stellt die Zugabe von $Ca^{2+}$-Ionen zur Messlösung dar. Dabei wird, nach Aufsetzen eines Vesikels auf die Apertur die $Ca^{2+}$-Konzentration auf > 2 mM gesteigert. Das sofortige elektrische Abdichten der Membran wird in Figur 11 gezeigt.

Es hat sich gezeigt, dass ein Aufbau mit einer kurzen Distanz (D < 5 mm) zwischen den Elektroden vorteilhaft ist, um bei kleinen Spannungen ($V_c$ < 200 mV, insbesondere -200 bis 200 mV) eine hohe Feldstärke für die elektrophoretische Positionierung der Vesikel oder Zellen zu erreichen.

Die Elektroden werden vorzugsweise in einem solchen Abstand zueinander angebracht und die Kompartimente sowie die Apertur derart mit Puffern resp. Lösungen gefüllt werden, dass sich in einem sphärisch, aber beliebig geformten in die Kompartimentflüssigkeit hineinreichenden Raumgebiet um die Apertur Feldstärken > 100 V/m ergeben.

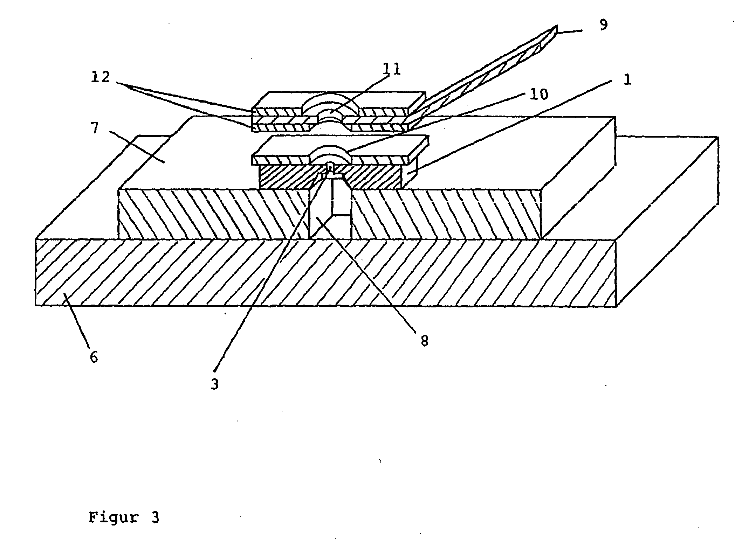

Im folgenden wird der konkrete Aufbau eines durch die Erfindung ermöglichten Messystems mit planaren Elektroden beschrieben (Fig. 3): Eine Elektrode 6, beispielsweise eine Silberplatte (z.B. Reinheit >99.98% Ag, geringere Reinheit ist aber auch möglich)mit den Abmessungen 20 x 20 x 2 mm³, wird als Sensorträger und gleichzeitig als Referenzelektrode verwendet. Auf dieser Elektrode ist, mittels eines Abstandhalters 7, z.B. einer 0.5-2 mm dicken Silikonkautschuk Dichtung (Sylgard 184, Dow Corning, USA) im richtigen Abstand und parallel, der eigentliche Membranträgerchip 1 positioniert. Der Abstandhalter weist einen ca. 1 mm breiten und < 6 mm langen Kanal (bzw. eine Kammer) 8 auf, der beispielsweise eingeprägt werden kann und der, mit Pufferlösung gefüllt, Kontakt zwischen der Apertur 3 resp. der Membran und der Referenzelektrode 6 herstellt. Zur Herstellung einer Referenzelektrode, z.B. der bevorzugten Ag/AgCl Elektrode, wird der Kanal beispielsweise mit 1 M HCl gefüllt und unter Spannung, z.B. bei V = 0.8 V das im Kanal exponierte Silber für 90 Sek. chloriert. Die Trägerchips 1 werden nach Benetzung ihrer Unterseite mit Pufferlösung auf den mit Referenzpufferlösung gefüllten Kanal 8 aufgesetzt.

Bei der Messung bzw. der Membranherstellung wird die Mess- bzw. Vesikellösung dann direkt auf die Apertur 3 resp. das Fenster 4 auf der Oberseite des Trägerchips 1 bzw. auf die Oberseite der Messelektrode 9 gegeben, z.B. ein Volumen V von ca. 5-10 µl. Zur Minimierung von Störanfälligkeiten kann die Region um die Apertur 3 in einem Abstand von z.B. r = 1 mm durch einen Silikonring 10 (Sylgaard) begrenzt werden. Dieser Ring 10 bildet zusammen mit dem Meniskus, der sich zwischen Chip und Messelektrode ausbildet, die Probenkammer (Probenkompartiment). Die Messelektrode 9, beispielsweise aus 0.8 mm starkem chloriertem quadratischem Silberblech (z.B. 4x4 mm²),insbesondere aber ringförmigem Silberblech (z.B. d = 2 mm) wird insbesondere parallel zur Chipoberfläche positioniert, vorzugsweise in einem Abstand bis zu ca. 1 mm, wobei sich die zur Zell- resp. Vesikel- und Messlösungszugabe darin befindliche und vorzugsweise trichterförmige Öffnung 11 ($d_{min}$ = 0.4-1 mm) vorzugsweise genau konzentrisch gegenüber der Mikroapertur 3 im Trägerchip 1 befindet. Die Messelektrode kann oberhalb und unterhalb mit je einem Abstandhalter 12 versehen sein, die zur Gestaltung der Zugabeöffnung 11 beitragen.

Durch Ausnutzung von Kapillarkräften bei der Füllung und Speicherung des Referenz- und Messpuffers ist das System trotz seiner Offenheit bezüglich der Flüssigkeitsspeicherung mechanisch sehr stabil. Durch die Offenheit werden Störungen der Membran durch hydrostatische Druckdifferenzen, wie sie bei geschlossenen Systemen z.B. durch Temperaturdifferenzen auftreten können, ausgeschlossen.

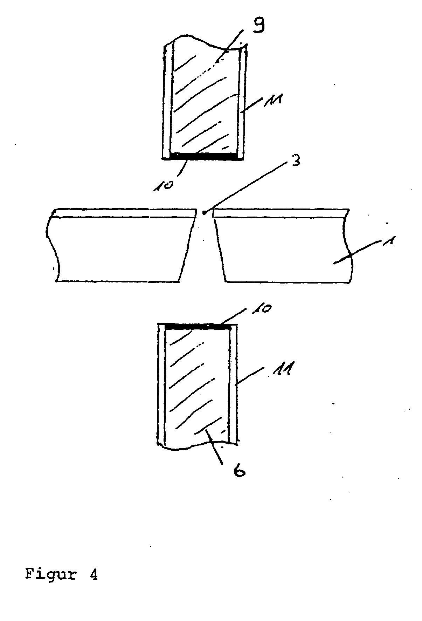

Eine weitere Realisierungsvariante verwendet Punkt- bzw. Drahtelektroden (Fig. 4): Dabei wird der oberflächenmodifizierte und gegebenenfalls auf einem insbesondere planaren Halter, z.B. einem Glas- oder Teflonhalter befestigte Chip 1 zwischen die chlorierten Endflächen 10 zweier oberhalb und unterhalb des Chips 1 angeordneter, gegebenenfalls aussen (mit Ausnahme der Endflächen) mit einer Schutzschicht 11, insbesondere einer Teflonschicht, versehenen Silberdrähte, resp. Silberelektroden 6, 9 mit beispielsweise einem Durchmesser d = 0.1 - 2 mm (ohne Schutzschicht) gebracht (Distanz Elektrode 6 - Elektrode 9 z.B. 4 mm). Mittels Pipette oder nahe am Chip angebrachten Schläuchen oder einem der im folgenden aufgezählten Probenhandhabungssysteme wird Pufferlösung zu beiden Seiten des Chips gegeben und durch Kapillarkräfte zwischen Chip und Elektrode gehalten. Nach Offsetkalibrierung und Anlegen einer geeigneten Spannung von üblicherweise V = -60 bis - 100 mV werden die Vesikel/Zellen mittels Pipette oder eines weiteren Schlauchs oder einem der im folgenden aufgezählten Probenhandhabungssysteme zur modifizierten Chipseite hinzugegeben. Vesikelbindung und Membranausbildung werden anhand der Veränderung der elektrischen Parameter verfolgt.

Die allgemein beschriebene Messanordnung sowie die im Detail beschriebenen Realisierungsbeispiele eignen sich prinzipiell für integrierte und um ein Probenhandhabungssystem erweiterte Systeme. Dazu zählen flüssigkeitstransportierende Systeme die z.B. auf der Basis von Pumpen, hydrostatischen Druckdifferenzen, elektroosmotischen, piezoelektrischen und Temperatur Effekten oder mechanischer Verschiebung definierte Flüssigkeitsvolumina in die und/oder aus den Flüssigkeitskompartimenten des beschriebenen Aufbaus befördern können. Gleichzeitig ist eine einfache Parallelisierung des beschriebenen Aufbaus, entweder auf einem Multiaperturchip oder mit mehreren Trägerchips mit einer Apertur, möglich.

Grosse unilamellare Vesikel (Giant Unilamellar Vesicles, GUV), die als Membranen geeignet sind, können mittels der Hydratationsmethode hergestellt werden (H.H. Hub, U. Zimmermann et al. (1982). "Preparation of large unilamellar vesicles." FEBS Lett. 140(2): 254-256; P. Mueller, T.F. Chien et al. (1983). "Formation and properties of cell-size lipid bilayer vesicles." Biophys. J. 44 (3): 375-81; K. Akashi, H. Miyata et al. (1996). "Preparation of giant liposomes in physiological conditions and their characterization under an optical microscope." Biophys. J. 71(6): 3242-50). Diese Methode gestattet bei entsprechender Modifizierung auch die Herstellung von Proteoliposomen (M. Criado und B.U. Keller (1987). "A membrane fusion strategy for single-channel-recordings of membranes usually non-accessible to patch-clamp pipette electrodes." FEBS Lett. 224(1): 172-6; B.U. Keller, R. Hedrich et al. (1988). "Single channel recordings of reconstituted ion channel proteins: an improved technique." Pflugers Arch 411(1): 94-100).

Um eine elektrisch dichte Bindung der Vesikel an den Chip zu gewährleisten, ist eine der Trägeroberfläche entgegengesetzte Nettoladung der Vesikeloberfläche erforderlich. Die Vesikeloberfläche kann z.B. durch Palmitoyloleylphosphatidylglycerol (POPG), negativ geladen werden, um möglichst: physiologische Verhältnisse für membraninkorporierte Proteine zu gewährleisten.

Nach der Positionierung können Zellen resp. Vesikel, falls diese nicht von selbst platzen, z.B. durch Behandlung mit hypotonischem Medium, z.B. reinem Wasser, aufgebrochen werden.

Messaufbauten des planaren Typs eignen sich durch die damit verknüpften kurzen Diffusionszeiten auch besonders für die Verwendung von 'Perforated Patch' Techniken (R. Horn und A. Marty (1988). "Muscarinic activation of ionic currents measured by a new whole-cell recording method." J. Gen. Physiol. 92(2): 145-59; J. Rae, K. Cooper et al. (1991). "Low access resistance perforated patch recordings using amphotericin B." J. Neurosci. Methods. 37(1): 15-26). Bei diesen Techniken erreicht man eine elektrische Verbindung zum Zellinnern (Cytosol) durch Permeabilisierung des normalerweise an der Glaspipette haftenden Membranfleckens mit porenausbildenden Antibiotika. Ein Vorzug dieser Technik ist das Nichtauswaschen des Cytosols mit Messpufferlösung bei gleichzeitigem elektrischen Zugang.

Erfindungsgemäss kann ein Porenbildner wie z.B. Amphotericin B oder Nystatin in das Referenzkompartiment gegeben werden, nachdem eine biologische Zelle oder unter speziellen Umständen auch Vesikel (wobei deren mechanische Stabilität ausreichend hoch sein muss) an die Aperturoberseite gebunden wurden. Dabei ist die Geschwindigkeit der Perforierung des Membranpatches über der Apertur wesentlich grösser als bei vergleichbaren Patch-clamp-Techniken.

Zudem ist auch bei Zerstörung des Membranpatches, analog der Whole-Cell-Patch-Clamp-technik (WCRT), eine einfache Zugabe grösserer Proteine über die Referenzlösung in das Cytoplasma möglich. Grund dafür ist der planare Aufbau des Messsystems, welcher im Vergleich zur WCRT eine wesentlich schnellere Diffusion grosser Makromoleküle in das Cytosol bzw. Vesikelinnere erlaubt (Z.M. Pei, J.M. Ward et al. (1996). "A novel chloride channel in Vicia faba guard cell vacuoles activated by the serine/threonine kinase, CDPK." EMBO J. 15(23): 6564-74).

Die durch die Erfindung ermöglichte Messanordnung resp. das erfindungsgemässe Positionierverfahren haben sehr breite Anwendungsmöglichkeiten. Neben den bereits vorne diskutierten Verwendungsmöglichkeiten können sie noch zur Trennung resp. zur Grössenanalyse von Vesikeln resp. Zellen dienen, der Positionierung von Zellen für z.B. rein optische Untersuchungen oder Mikroinjektionen. Das System gestattet insbesondere die direkte funktionelle Analyse ionotroper Membranproteine, z.B. in Ligandenbindungsstudien. In Verbindung mit seinem einfachen Aufbau der Reproduzierbarkeit der Ergebnisse und der mechanischen Stabilität der Membranabschlüsse eignet es sich auch besonders für Biosensoren im Screening-Bereich und stellt hier eine kosten- und zeitsparende Alternative zu Patch-clamp-Techniken dar. Die einfache Parallelisierbarkeit des Systems macht es prinzipiell auch für HTS geeignet.

Die Untersuchungen können unter Verwendung von Zellen und Vesikeln aber auch Zellfragmenten, zellulärer Organellen und Lipidmembranen erfolgen.

Das Verfahren gestattet eine Aufzeichnung des Membranwiderstandes mit gutem Signal-Rauschabstand.

Im erfindungsgemässen Verfahren können die Messlösung oder die Referenzlösung oder beide Lösungen durch eine andere Lösung ausgetauscht werden oder eine zu analysierende Substanz zur Lösung mess- und/oder referenzseitig hinzugegebenwerden, z.B. ein Porenbildner, der einem oder beiden Kompartimenten zugesetzt werden kann mit dem Ziel, die elektrische Leitfähigkeit resp. die Permeabilität der Membran gegenüber bestimmten Ionen zu erhöhen, oder Proteoliposomen beliebiger Grösse, mit dem Ziel, diese mit der Membran über der Apertur 3 zu fusionieren und damit beliebige darin enthaltene Membranproteine elektrischen oder optischen Messungen zugänglich zu machen. Auch können nach dem Aufbau einer Membran über der Apertur (3) Membranproteine in diese eingebaut werden.

Das Verfahren kann auch derart durchgeführt werden, dass eine Apparatur verwendet wird, die derart gestaltet ist, dass die über der Apertur 3 befindliche Membran optischen, insbesondere Fluoreszenzmessungen, zugänglich ist und diese daran ausgeführt werden.

Auch können mehreren Aperturen 3 auf einem Träger verwendet werden, und die Messungen über mindestens zwei Aperturen 3 sequentiell und/oder parallel erfolgen und/oder derart, dass alle oder mehrere Elektroden auf einer Seite des Trägers 1 ein gemeinsames elektrisches Potential besitzen resp. zu einer Elektrode vereinigt werden.

Das Verfahren wird nun anhand von Beispielen näher erläutert. Diese sollen in keiner Weise als den Umfang der Erfindung einschränkend ausgelegt werden.

Beispiele

Vesikel-Bildung und Grössentrennung

100 μl Asolectin (Fluka) oder Eilecithin (EPC), 50 μl Palmitoyloleylphosphatidylglycerol (POPG), 3 μl Dipalmitoylphosphotidylethanolamin-Rhodamin (DPPE-Rhodamin) (Molecular Probes, USA) (alles 10 mg/ml in Chloroform, Avanti Polar Lipids) und 70 μl Methanol wurden in einem Rotationsverdampfer (Büchi Rotavapor R-114) bei 400 mmHg Unterdruck in einem 10 ml Rundkolben zu einem Film eingetrocknet. Nach anschliessender einstündiger Inkubation im Vakuum wurden 10 ml $H_2O$ oder 10 ml eine Pufferlösung mit < 150 mM KCl und/oder < 600 mM Sucrose oder vorzugsweise Sorbitol hinzugegeben. Die bei diesem Vorgang gebildeten Vesikel zeigten sich als fast durchsichtige Wolke nach etwa 16 h bei 37°C. Die Vesikel wurden mit einer 1 ml Pipette abgesaugt und nach (optionaler) Zugabe von Natriumazid ($NaN_3$, Endkonzentration 0,2 Gew.-%) bei 4°C bis zur weiteren Verwendung gelagert.

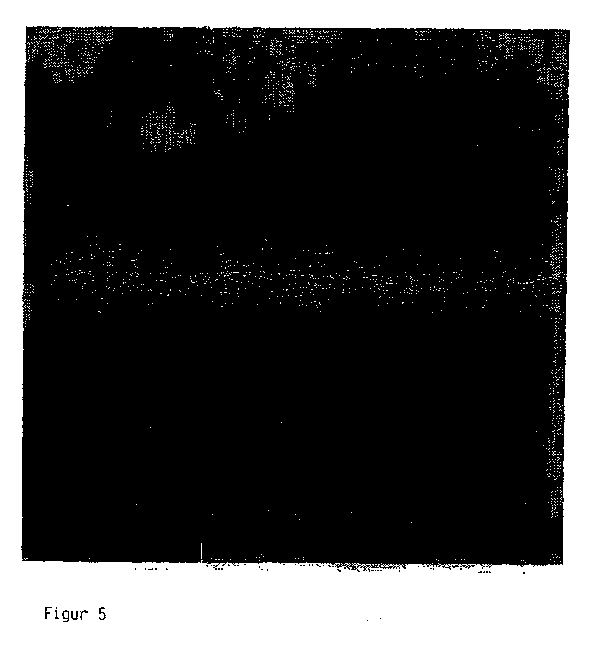

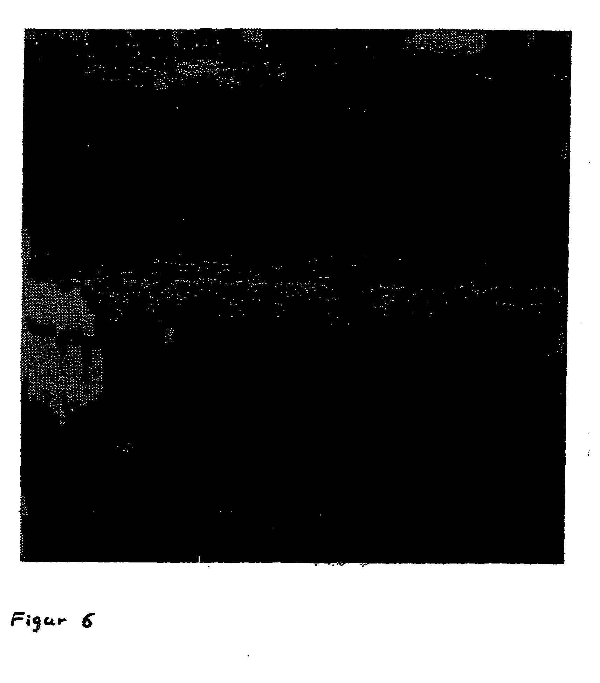

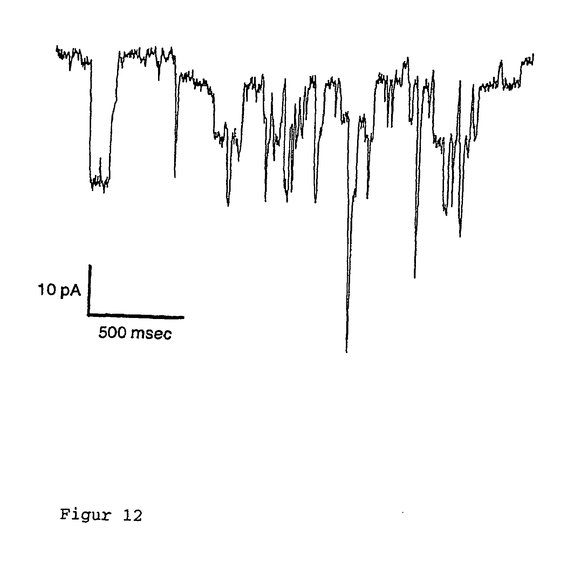

Die Herstellung von Lipidvesikeln nach diesen Verfahren ergab grösstenteils (> 90 %) unilamellare Vesikel bis zu einer Grösse von 250 μm (Fig. 5). Ein Teil der Vesikel enthielt weitere, kleinere Vesikel, die aber für die Membranbildung nicht relevant waren. Die Verwendung von gereinigten Vesikeln zum Aufbau elektrisch dicht mit der Oberfläche abschliessender Membranen erforderte die Abtrennung aller Vesikel und Lipidverunreinigungen, die kleiner als 10 μm waren. Bei unzureichender Trennung kam es durch die Bindung kleiner Vesikel in Aperturnähe zu Abstossungseffekten, die einen elektrisch dichten Aperturabschluss durch grosse Vesikel (> 10 μm) verhinderten. Die Vesikel wurden bezüglich ihrer Grösse durch > 20 h Dialyse über ein Nylonnetz mit 20 μm Porengrösse getrennt; wenn nötig kann bei entsprechender Lipidzusammensetzung die Fluidität der Vesikelmembran erniedrigt werden durch Absenkung der Temperatur auf < 4°C, insbesondere 1°C. Die Unilamellarität der Vesikelmembranen kann bei geeigneter Präparation durch Zugabe von Alamethicin (siehe dazu R.B. Gennis (1989). Biomembranes: molecular structcure and function. New York, Berlin, Heidelberg, Springer Verlag) gezeigt (Fig. 12) und durch konfokalmikroskopische Analysen gestützt werden (Fig. 6).

Elektrophoretische Positionierung der Vesikel

Variante 1:

Vor jeder Messung wurde die Offsetspannung $V_{offset}$ zwischen den Elektroden korrigiert. Dazu wurden 5 μl Pufferlösung direkt auf die Apertur gegeben und anschliessend die Messelektrode bis auf 1 mm an die Chipoberfläche genähert. Nach der Ausbildung eines Flüssigkeitsmeniskus zwischen Chipoberfläche und Elektrode wurden Offsetspannung und Kapazität des Systems kompensiert.

10 μl einer vesikelhaltigen Dispersion wurden anschliessend auf die Oberseite der Messelektrode gegeben, wobei die Vesikel durch die in der Messelektrode befindliche kreisförmige Oeffnung sedimentieren konnten. Vesikel, die sich durch die Messelektrodenöffnung bewegt hatten, wurden unter dem Einfluss des der angelegten Elektrodenspannung VM = -50 bis -80 mV entsprechenden elektrischen Feldes direkt auf die Apertur beschleunigt. Dabei war die erreichte Fokussierung, gemessen an der Zahl von Vesikeldurchtritten durch die Apertur bei unmodifizierten Oberflächen, von der Fenstergrösse abhängig (als Fenster wird der durch das Aetzen freigelegte Teil der $SiO_2$ Schicht bezeichnet (Fig. 2). In Gegenwart kleiner $SiO_2$ Fenster (< 45 x 45 $\mu m^2$) war der Vesikeldurchsatz deutlich gesteigert.

Variante 2:

Vor jeder Messung wurde die Offsetspannung $V_{offset}$ zwischen den Elektroden korrigiert. Dazu wurde nach Zugabe von 5 μl Pufferlösung zwischen Chip und Messelektrode resp. Referenzelektrode die Spannung ermittelt, bei welcher der Stromfluss verschwindet, d.h. I ($V_{offset}$) = 0 pA.

3 μl einer vesikelhaltigen Dispersion wurden anschliessend zum Messkompartiment nahe der Apertur gegeben, wobei die Vesikel im Fall einer planparallelen Elektrodenanordnung durch die in der Messelektrode befindliche kreisförmige Öffnung sedimentieren konnten. Vesikel, die in den Bereich sehr hoher Feldstärke (bis zu mehreren kV) in der Nähe (< 200 μm) der Apertur kamen, wurden entsprechend des elektrischen Feldverlaufes direkt auf die Apertur beschleunigt und nach elektrisch dichter Bindung an die Chipoberfläche elektrisch analysiert.

Die in Variante 1 und 2 beschriebene elektrische Positionierung war der gleichfalls getesteten Schwerkraft-Sedimentation von Zellen und Vesikeln in folgenden Punkten überlegen: notwendige Vesikel- bzw. Zellanzahl, insgesamte Geschwindigkeit der Membranbildung und Wahrscheinlichkeit eines erfolgreichen Membranaufbaues bzw. Zellbindung.

Vesikel Bindung und Adsorption an modifizierte SiO$_2$-Oberflächen

Die Bindung der oben beschriebenen Vesikel an Polylysin-modifizierte SiO$_2$-Oberflächen wurde untersucht. Diese war sehr stark und ereignete sich nach entsprechender Annäherung in weniger als 0.5 sec. Die Wahrscheinlichkeit einer erfolgreichen und elektrisch dichten Positionierung war stark von der Aperturgrösse, der SiO$_2$ Fenstergrösse sowie der Anzahl, Grösse und Grössenverteilung der Vesikel abhängig. Trägerchips mit Aperturen $d_{Apertur}$ < 2 $\mu$m und Fenstern < 40 $\mu$m ergaben in Verbindung mit Suspensionen von Vesikeln $d_{Vesikel}$ > 40 $\mu$m eine Wahrscheinlichkeit > 90 % (n > 15, wobei n die Anzahl Versuche bezeichnet) elektrisch dichter Membranabschlüsse. Geringe Schwankungen in der Ausbildung dichter Aperturabschlüsse von Chip zu Chip und bei verschiedenen Vesikelsuspensionen lassen darauf schliessen, dass eine Steigerung nutzbarer Aperturabschlüsse bei weiterer Verkleinerung der Aperturweite sowie der Verwendung besser gereinigter Vesikelsuspensionen verzeichnet werden kann.

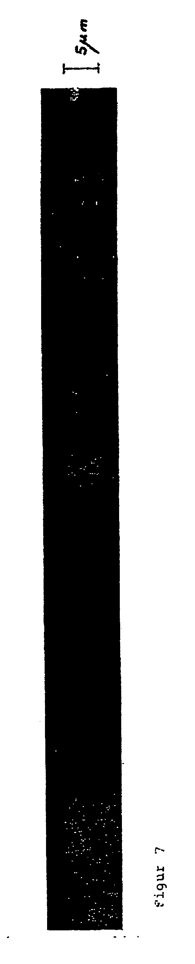

Bei der Bindung der Vesikel an der Oberfläche wurden diese zu völlig flachen Gebilden ausgezogen (Fig. 6 und 7). Die Membran der Vesikel wurde mit 0.5% Rhodamin markiert (rot) und das Vesikelinnere mit Carboxyfluorescein (grün). Das Verschwinden jeglicher Carboxyfluorescein-Emission (eine Farbaufnahme zeigt ausschliesslich die rote Farbe des Rhodamins, in Fig. 6 als grau der Membranflecken erkennbar) deutet auf die Freigabe von Carboxyfluorescein und damit auf das Bersten der Vesikel und mithin Unilamellarität dieser Membranen hin. Der dabei an den Trägerchips gemessene sehr hohe Membranabdichtwiderstand von R$_M$ > 6.4 G$\Omega$ (n = 26) in symmetrisch 85 mM KCl lässt auf die Ausbildung elektrisch weitgehend defektfreier Lipidmembranen schliessen. Der Fusionsprozess und das Fusionsergebnis der Vesikel auf Polylysin beschichtetem Glas wurden mit einem Konfokalmikroskop (LSM 510, Zeiss Jena, Germany) untersucht.

Bei einer analogen Messerie in symmetrisch 10 mM KCl wurde Bindung der Vesikel nach entsprechender Annäherung in weniger als 0.2 sec. mit einer Wahrscheinlichkeit von > 70 % (n >15) sowie ein Membranwiderstand von R$_M$ > 10 G$\Omega$ gemessen.

Elektrische Parameter der Lipidmembranen

Vor jeder Fusion eines Vesikels mit der modifizierten Oberfläche wurde der hauptsächlich durch die Apertur bestimmte Widerstand des Messaufbaus bestimmt. Dieser betrug je nach Aperturgrösse bis zu 1 M$\Omega$ (gewöhnlich < 450 k$\Omega$) in 85 mM KCl. Grössere Widerstände wurden als Artefakte, wie den Einschluss von Luftblasen unter der Apertur, gewertet.

Die bei der Vesikelfusion oder Zellbindung über der Apertur gebildeten Membranen besassen einen Widerstand R$_M$ > 6.4 G$\Omega$ bei gleicher Ionenkonzentration.

Dabei änderte sich die Kapazität der Trägerchips von einigen pF nur unwesentlich.

In einem analog durchgeführten Versuch in 1 mM KCl wurde der Widerstand des Messaufbaus ebenfalls als je nach Aperturgrösse bis zu üblicherweise 1 M$\Omega$ bestimmt. Bei gleicher Ionenkonzentration betrug der Widerstand der über der Apertur gebildeten Membranen üblicherweise R$_M$ > 40 G$\Omega$ und in 10 mM KCl üblicherweise RM > 10 G$\Omega$. Die Kapazität der Trägerchips änderte sich auch in diesen Versuchen nur unwesentlich, von 160 - 280 pF.

Vesikeldurchgang durch Mikrometerporen

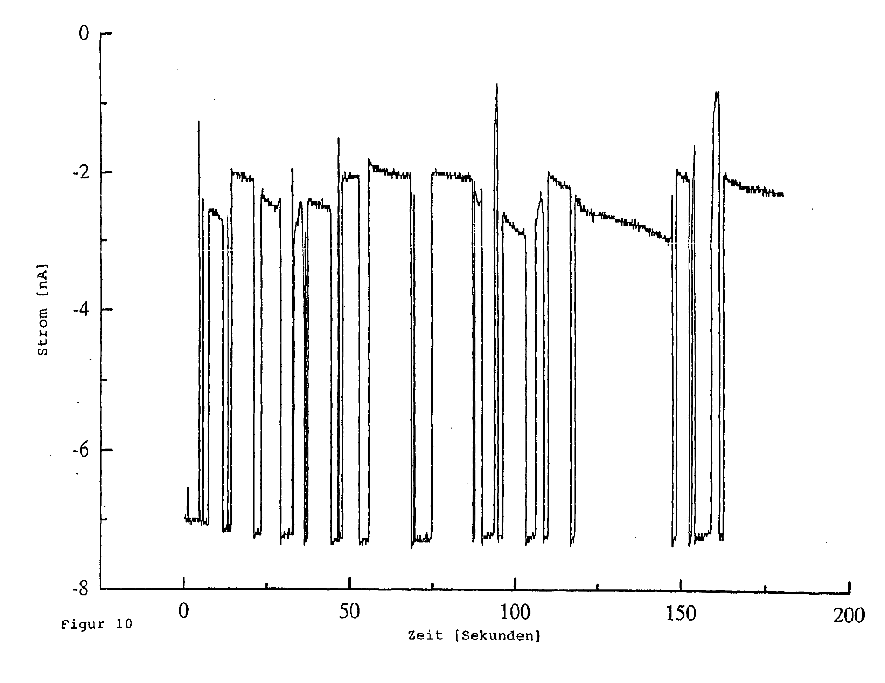

In Gegenwart negativ geladener Oberflächen, wie unmodifizierter SiO$_2$ Schichten oder um die Apertur fusionierter Vesikel, konnte der Durchgang von Vesikeln durch die Apertur anhand der Widerstandsänderungen beobachtet werden (Fig. 10). Zur Kontrolle auf Artefakte wurde die Spannung umgepolt, wobei keine Widerstandsmodulationen beobachtet wurden.

Bis zu 18 Sek. lange Vesikelpassagen lassen auf das Passieren sehr grosser Vesikel mit ausreichend fluiden Membranen schliessen. Besonders bei der Verwendung von Vesikelpopulationen mit d > 50 $\mu$m (n = 4, wobei n die Anzahl Messungen angibt) und einer Apertur d = 7 $\mu$m konnte eine fast ausschliessliche Variation der Durchgangszeit bei fixierter Amplitudenänderungen in Abhängigkeit der Vesikelgrösse beobachtet werden. Es kann darauf geschlossen werden, dass Vesikel bei ihrem Durchgang durch die Apertur zu schlauchartigen Gebilden mit definiertem Durchmesser und geschlossener Oberfläche ausgezogen werden. Die sich in der Schlauchlänge widerspiegelnde Grösse der Vesikel ist als Amplitudendauer zugänglich.

Damit lässt sich, durch Analyse der für grosse Vesikel ($d_{Vesikel}$ >> $d_{Apertur}$) typischen Durchgangszeit und der für kleine Vesikel ($d_{Vesikel}$ ~ $d_{Apertur}$) typischen Widerstandsamplitudenänderung, die grössenmässige Vesikelzusammensetzung einer Lösung bestimmen. Damit eröffnet dieses Verfahren ausserdem die Möglichkeit Populationen von Vesikeln und Zellen zu analysieren.

Beobachtung von Alamethicin Poren und nicotinischen Acetylcholinrezeptoren

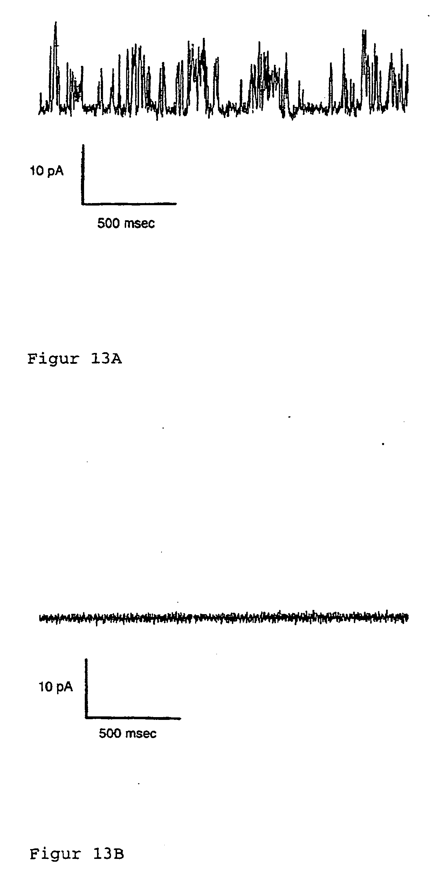

Zur Verifizierung der biologischen Funktionsweise des erfindungsgemässen Systems wurde, nach der Ausbildung einer Membran über der Apertur in 85 mM KCl, Alamethicin (Endkonzentration im Puffer 0.1 $\mu$g/ml) zum Messkopartiment gegeben(R.B. Gennis (1989). Biomembranes: molecular structure and function. New York, Berlin, Heidelberg, Springer Verlag). Das Auftreten der für Alamethicin typischen Stromfluktuationen (Amplituden und Verweilzeiten), denen Leitfähigkeiten der Alamethicin-Poren von etwa 600 pS entsprechen, beweist die Funktionsfähigkeit und hohe Sensitivität des Systems (Fig. 12). Gleichfalls können Rezeptorproteine, z.B. der nAChR, über die - z.B. $Ca^{2+}$ vermittelte - Fusion mit kleinen Vesikeln (small, medium and large unilammelare vesicles) in die Membran eingebracht und vermessen werden. Dazu wurde der nAChR gereinigt und entsprechend Schürholz in Vesikel rekonstituiert (Schürholz, T., J. Kehne, et al. (1992). "Functional reconstitution of the nicotinic acetylcholine receptor by CHAPS dialysis depends on the concentrations of salt, lipid, and protein." Biochemistry 31(21): 5067-77; Schürholz, T. (1996). "Critical dependence of the solubilization of lipid vesicles by the detergent CHAPS on the lipid composition. Functional reconstitution of the nicotinic acetylcholine receptor into preformed vesicles above the critical micellization concentration." Biophys Chem 58(1-2): 87-96). Diese Vesikel wurden zum Messkompartiment gegeben und durch Erhöhung der $Ca^{2+}$ Konzentration der Probenkammer auf > 1 mM und, gegebenenfalls unterstützt durch den anschliessenden temporären Aufbau eines osmotischen Gradienten (siehe: Eray, Dogan et al. 1995 a.a.O.), mit der Membran fusioniert. In Abwesenheit von Agonisten wurden typische Rezeptoröffnungsereignisse aufgezeichnet (Fig. 13A), die nach Zugabe von Carbamylcholin (20 μM Endkonzentration) innerhalb kurzer Zeit (t < 100 sec) weitgehend verschwanden (Fig. 13B, Desensitisierung).

Die Bindung von Zellen

Bei Substitution der Vesikel durch biologische Zellen lassen sich diese analog zu den verwendeten Vesikeln positionieren und elektrisch charakterisieren. Durch die Stützung der Zellmembran durch das Cytoskelett kommt es allerdings nicht automatisch zu einem Platzen der Zellen. Damit wird also nach Bindung der Zelle an die Chipoberfläche eine der Cell Attached Technik (CAT, Hamill, Marty et al. a.a.O., 1981) ähnliche Konfiguration erreicht. Voraussetzung für eine rauschfreie Messung ist hier vor allem eine relativ glatte Membranoberfläche. So ist z.B. bei Verwendung von Pflanzenzellen unbedingt die Zellwand zu entfernen.

Ausgehend von der CAT können, beispielsweise durch elektrische Zerstörung des Membranpatches über der Apertur, elektrische Messungen über die gesamte Zellmembran durchgeführt werden (Whole Cell Recording). Die Zugabe von Porenbildnern, beispielsweise Amphotericin B oder Nystatin, zum Referenzkompartiment und folglich Permeabilisierung des Membranpatches über der Apertur kann ebenfalls für Ganzzellmessungen verwendet werden (Perforated Patch Technik).

Weiterhin ist, durch Lysis der Zelle, die Aufzeichnung von Einzelkanal Ereignissen in der sogenannten Inside-Out Konfiguration möglich, bei der die cytosolische Membranseite der Messlösung ausgesetzt ist.

Patentansprüche

1. Verfahren zur Positionierung von Membranen in Form von Zellen oder Vesikeln oder anderer biologischer Organellen und zum elektrisch dichten Binden von Vesikeln, Zellen, oder anderen biologischen Organellen, resp. Membranen entsprechenden Ursprungs an einer Apertur mittels einer Messanordnung mit mindestens zwei Elektroden (6, 9) und separierten, für die Aufnahme von Flüssigkeit geeigneten Kompartimenten, wobei sich zwischen zwei gegenüber befindlichen, in je mindestens ein Kompartiment hineinreichenden oder ein solches berührenden und beliebig geformten Redoxelektroden (6, 9) ein elektrisch isolierender Träger (1) befindet, der jeweils mindestens 2 Kompartimente voneinander trennt und der mindestens eine Apertur (3) enthält, wobei die Apertur (3) die Kompartimente verbindet und einen solchen Durchmesser aufweist, dass sich bei Existenz einer Spannungsdifferenz über dem Träger, vermittelt durch die Elektroden (6, 9), ein inhomogenes elektrisches Feld um die Apertur aufbaut, das mit Annäherung an die Apertur betragsmäßig größer wird und nahe der Apertur Vesikel, Zellen, Zellfragmente oder biologische Organellen elektrophoretisch auf diese zubewegen kann, was genaue Positionierung ermöglicht, und wobei elektrisch dichte Bindung von Vesikeln, Zellen und anderen biologischen Organellen, resp. organischen Membranen entsprechenden Ursprungs, an der Apertur ermöglicht wird, **dadurch gekennzeichnet, dass** Zellen resp. Vesikel oder andere biologische Organellen in den vorher mit Puffer gefüllten oder ungefüllten Zwischenraum zwischen Trennwand resp. Träger und Elektrode gegeben werden und zwischen beiden Elektroden eine elektrische Spannungsdifferenz, in einem Bereich von -200 mV bis +200 mV, besteht, die zur Ausbildung eines inhomogenen elektrischen Feldes führt, und dass die Elektroden in einem solchen Abstand zueinander angebracht und die Kompartimente sowie die Apertur derart mit Puffern resp. Lösungen gefüllt werden, dass sich in einem sphärisch, aber beliebig geformten in die Kompartimentflüssigkeit hineinreichenden Raumgebiet um die Apertur Feldstärken > 100 V/m ergeben, unter deren Einfluss eine gerichtete Bewegung der Vesikel oder Zellen auf die Apertur stattfindet und die Zellen resp. Vesikel oder andere biologische Organellen durch eine starke elektrostatische Anziehung zwischen der Trägeroberfläche und der Membranoberfläche eine elektrische dichte Bindung eingehen.

2. Verfahren zur elektrischen Analyse von natürlichen oder künstlichen Lipidmembranen, Vesikeln, Zellen oder biologischen Organellen, **dadurch gekennzeichnet, dass** die Membranen mittels des Verfahrens gemäß Anspruch 1 positioniert werden, mit der Oberfläche (5) des Trägers (1) eine elektrisch dichte Verbindung über der Apertur (3) eingehen und eine Aufzeichnung des Membranwiderstandes mit gutem Signal-Rauschabstand gestatten.

3. Verfahren zur elektrischen Messung von Interaktionen an oder in natürlichen oder künstlichen Lipidmembranen, Vesikeln, Zellen oder biologischen Organellen, **dadurch gekennzeichnet, dass** die Membranen gemäß Anspruch 1 erzeugt werden und dass die Messlösung oder die Referenzlösung oder beide Lösungen durch eine andere Lösung ausgetauscht werden oder eine zu analysierende Substanz zur Lösung mess- und/oder referenzseitig hinzugegeben wird.

4. Verfahren gemäß Anspruch 2 oder 3, **dadurch gekennzeichnet, dass** ein Porenbildner einem oder beiden Kompartimenten zugesetzt wird mit dem Ziel, die elektrische Leitfähigkeit resp. die Permeabilität der Membran gegenüber bestimmten Ionen zu erhöhen.

5. Verfahren gemäß einem der Ansprüche 2 bis 4, **dadurch gekennzeichnet, dass** zu mindestens einem Kompartiment Proteoliposomen beliebiger Größe gegeben werden mit dem Ziel, diese mit der Membran über der Apertur (3) zu fusionieren und damit beliebige darin enthaltene Membranproteine elektrischen oder optischen Messungen zugänglich zu machen.

6. Verfahren gemäß einem der Ansprüche 2 bis 5, **dadurch gekennzeichnet, dass** Membranproteine nach dem Aufbau einer Membran über der Apertur (3) in diese eingebaut werden.

7. Verfahren gemäß einem der Ansprüche 2 bis 6, **dadurch gekennzeichnet, dass** die über der Apertur (3) befindliche Membran optischen, insbesondere Fluoreszenzmessungen, zugänglich ist und diese daran ausgeführt werden.

8. Verfahren gemäß einem der Ansprüche 2 bis 7, **dadurch gekennzeichnet, dass** eine Messanordnung oder ein Messsystem mit mehreren Aperturen (3) auf einem Träger verwendet wird, und dass Messungen über mindestens zwei Aperturen (3) sequentiell und/oder parallel erfolgen.

9. Verfahren gemäß Anspruch 8, **dadurch gekennzeichnet, dass** alle Elektroden auf einer Seite des Trägers (1) ein gemeinsames elektrisches Potential besitzen resp. zu einer Elektrode vereinigt werden.

10. Verfahren gemäß einem der Ansprüche 2 bis 9, **dadurch gekennzeichnet, dass** mindestens eine Apertur der Messung verschiedener Membranparameter dienen kann, und dass mittels eines Pumpensystems, das über Schläuche mit beliebigen Kompartimenten verbunden ist, oder mittels eines Verfahrens auf hydrostatischer Druckbasis oder mittels eines Piezotropfenverfahrens resp. Tintenstrahlverfahrens oder mittels eines Kontakttransferverfahrens oder elektroosmotischen Verfahrens oder temperaturkontrollierten Verfahrens Flüssigkeiten resp. Proben in beliebigen Kompartimenten zugegeben oder ausgetauscht werden.

11. Verfahren gemäß einem der Ansprüche 2 bis 10, **dadurch gekennzeichnet, dass** es direkt gekoppelt mit einem Probenauftrennungsprozess, insbesondere Kapillarelektrophorese (CE) und HPLC, durchgeführt wird und der Analyse der aufgetrennten Substanzen dient.

12. Verfahren gemäß einem der Ansprüche 2 bis 11, **dadurch gekennzeichnet, dass** der Flüssigkeitsstand in den Kompartimenten kontinuierlich oder regelmäßig geprüft und entsprechend voreingestellten Füllparametern nachgeregelt wird.

13. Verfahren gemäß einem der Ansprüche 2 bis 12, **dadurch gekennzeichnet, dass** die Oberfläche (5) des Trägers (1) derart strukturiert wird, dass sich hydrophile und hydrophobe Bereiche ergeben, wobei sich die hydrophilen Bereiche vorzugsweise um die Apertur befinden.

## Claims

1. Method for the positioning of membranes in the form of cells or vesicles or other biological organelles and for electrical tight bonding of vesicles, cells or other biological organelles or membranes, respectively, of corresponding origin on the aperture by means of a measuring arrangement with at least two electrodes (6, 9) and separated compartments suitable for the uptake of liquid, wherein between two opposite arbitrarily formed redox electrodes (6, 9) each one protruding in or contacting at least one compartment, there is an electrically insulating carrier (1) which separates at least 2 compartments from one another and contains at least one aperture (3), wherein the aperture (3) connects the compartments and has such a diameter that, in case of a voltage difference over the carrier mediated by the electrodes (6, 9), an inhomogeneous electric field is built up around the aperture, which becomes larger in absolute value when approaching the aperture and can electrophoretically move near the aperture and towards it vesicles, cells, cell fragments or biological organelles, which makes accurate positioning possible, and wherein an electrically insulating bonding of vesicles, cells, and other biological organelles or organic membranes, respectively, of corresponding origin onto the aperture is made possible, **characterized in that** cells or vesicles or other biological organelles are introduced into the intermediate space between the separating wall or the carrier and electrode, respectively, previously filled with buffer or unfilled and an electrical voltage difference, in the range of -200 mV to + 200 mV, is applied between both electrodes, the voltage difference leads to the development of an inhomogeneous electric field, and that the electrodes are brought to such a distance from one another and the compartments as well as the aperture are filled with buffers or solutions in such a way that in a spherical but arbitrarily formed space penetrating into the compartment liquid around the aperture, field strengths of > 100 V/m develop, under the influence of which a directed movement of the vesicles or cells towards the aperture takes place, and the cells or vesicles or other biological organelles are engaged in an electrically tight bonding by a strong electrostatic attraction between the carrier surface and the membrane surface.

2. Method for the electrical analysis of natural or artificial lipid membranes, vesickles, cells or biological orgenelles, **characterized in that** the membranes are positioned using the method according to Claim 1, are brought with the surface (5) of the carrier (1) into an electrically insulating bonding above the aperture (3) and permit a recording of the membrane resistance with good signal-to-noise ratio.

3. Method for the electrical measurement of interactions on or in natural or artificial lipid membranes, vesicles, cells, or biological organelles, **characterized in that** the membranes are produced according to Claim 1 and that the measuring solution or the reference solution or both solutions are replaced by another solution or that a substance to be analysed is added to the solution on the measuring and/or reference side.

4. Method according to Claim 2 or 3, **characterized in that** a pore former is added to one or both compartments with the goal to increase the electrical conductivity or permeability, respectively, of the membrane with respect to certain ions.

5. Method according to one of the Claims 2 to 4, **characterized in that** proteoliposomes of arbitrary size are added to at least one compartment with the purpose of fusing them with the membrane above the aperture (3) and thus to make any arbitrary membrane proteins contained therein accessible to electrical or optical measurements.

6. Method according to one of the Claims 2 to 5, **characterized in that** after the building up of a membrane above the aperture (3), membrane proteins are incorporated into it.

7. Method according to one of the Claims 2 to 6, **characterized in that** the membrane located above the aperture (3) is accessible to optical, in particular fluorescence measurements and that these are carried out on it.

8. Method according to one of the Claims 2 to 7, **characterized in that** a measuring arrangement or a measuring system with several apertures (3) on a carrier is used and that measurements are carried out sequentially and/or parallel over at least two apertures (3).

9. Method according to Claim 8, **characterized in that** all electrodes on one side of the carrier (1) have a common electrical potential or are combined to one electrode, respectively.

10. Method according to one of the Claims 2 to 9, **characterized in that** at least one aperture can serve for the measurement of various membrane parameters and that, by means of a pump system, which is connected with tubes to arbitrary compartments or by means of a method based on hydrostatic pressure or by means of a piezo drop method or ink jet method, respectively, or by means of a contact transfer method or electro-osmotic method or temperature-controlled method, liquids or samples are added to or exchanged in arbitrary compartments.

11. Method according to one of the Claims 2 to 10, **characterized in that** it is performed directly coupled with a sample separation process, in particular capillary electrophoresis (CE) and HPLC, and that it serves for the analysis of the separated substances.

12. Method according to one of the Claims 2 to 11, **characterized in that** the liquid level in the compartments is checked continuously or regularly and is readjusted corresponding to predetermined filling parameters.

13. Method according to one of the Claims 2 to 12, **characterized in that** the surface (5) of the carrier (1) is structured in such a way that hydrophilic and hydrophobic regions are obtained wherein the hydrophilic regions are located preferably around the aperture.

**Revendications**

1. Procédé pour le positionnement de membranes en forme de cellules ou de vésicules ou d'autres organites biologiques et pour la liaison électrique étanche de vésicules, cellules ou autres organites biologiques et/ou membranes d'origine correspondante au niveau d'une ouverture au moyen d'un montage de mesure comprenant au moins deux électrodes (6, 9) et des compartiments séparés, adaptés à recevoir du liquide, un support (1) isolant électriquement se situant entre deux électrodes redox (6, 9) en vis-à-vis, qui pénètrent dans au moins un compartiment respectif ou sont au contact d'un tel compartiment et sont de forme quelconque, lequel support sépare respectivement au moins deux compartiments l'un de l'autre et comporte au moins une ouverture (3), l'ouverture (3) reliant les compartiments et présentant un diamètre tel qu'il s'établit autour de l'ouverture, en présence d'une différence de tension sur le support, transmise par les électrodes (6, 9), un champ électrique hétérogène qui s'élève à l'approche de l'ouverture et que des vésicules, cellules, fragments cellulaires ou organites biologiques peuvent se déplacer par électrophorèse sur l'ouverture au voisinage de cette dernière, ce qui permet un positionnement exact, et la liaison électrique étanche de vésicules, cellules et autres organites biologiques et/ou de membranes organiques d'origine correspondante, étant permise sur l'ouverture, **caractérisé en ce que** des cellules et/ou vésicules ou autres organes biologiques sont placés dans l'espace intermédiaire, préalablement rempli ou non rempli de tampon, entre la cloison et/ou le support et l'électrode et il existe entre les deux électrodes une différence de tension électrique, dans une plage de - 200 mV à + 200 mV, qui provoque la formation d'un champ électrique hétérogène, et que les électrodes sont montées à une distance mutuelle telle et les compartiments ainsi que l'ouverture sont remplis de tampons et/ou de solutions de telle sorte qu'il en résulte autour de l'ouverture, dans une région spatiale de forme sphérique, mais quelconque, qui pénètre dans le liquide des compartiments, des intensités de champ > 100 V/m sous l'effet desquelles se produit un déplacement dirigé des vésicules ou cellules sur l'ouverture et les cellules et/ou vésicules ou autres organites biologiques entrent en liaison électrique étanche par une forte attraction électrostatique entre la surface du support et la surface de la membrane.

2. Procédé d'analyse électrique de membranes lipidiques, vésicules, cellules ou organites biologiques, naturels ou artificiels, **caractérisé en ce que** les membranes sont positionnées au moyen du procédé selon la revendication 1, entrent en liaison électrique étanche sur l'ouverture (3) avec la surface (5) du support (1) et permettent un enregistrement de la résistance des membranes avec une bonne distance signal - bruit.

3. Procédé de mesure électrique d'interactions sur ou dans des membranes lipidiques, vésicules, cellules ou organites biologiques, naturels ou artificiels, **caractérisé en ce que** les membranes sont produites selon la revendication 1 et que la solution de mesure ou la solution de référence ou les deux solutions sont remplacées par une autre solution ou une substance à analyser est ajoutée à la solution du côté mesure et/ou du côté référence.

4. Procédé selon l'une des revendications 2 ou 3, **caractérisé en ce qu'**un agent porogène est ajouté à un ou aux deux compartiments dans le but d'élever la conductivité électrique et/ou la perméabilité de la membrane par rapport à des ions définis.

5. Procédé selon l'une des revendications 2 à 4, **caractérisé en ce que** des protéoliposomes de grosseur quelconque sont ajoutés à au moins un compartiment dans le but de les fusionner avec la membrane sur l'ouverture (3) et de rendre ainsi accessibles à des mesures électriques ou optiques des protéines membranaires quelconques qui y sont renfermées.

6. Procédé selon l'une des revendications 2 à 5, **caractérisé en ce que** des protéines membranaires, après la constitution d'une membrane sur l'ouverture (3), sont incorporées dans cette dernière.

7. Procédé selon l'une des revendications 2 à 6, **caractérisé en ce que** la membrane située sur l'ouverture (3) est accessible à des mesures optiques, en particulier des mesures de fluorescence, et ces dernières sont alors réalisées.

8. Procédé selon l'une des revendications 2 à 7, **caractérisé par** l'utilisation d'un montage de mesure ou d'un système de mesure avec plusieurs ouvertures (3) sur un support, et par la réalisation séquentielle et/ou parallèle de mesures sur au moins deux ouvertures (3).

9. Procédé selon la revendication 8, **caractérisé en ce que** toutes les électrodes présentent sur un côté du support (1) un potentiel électrique commun et/ou sont réunies en une électrode.

10. Procédé selon l'une des revendications 2 à 9, **caractérisé en ce qu'**au moins une ouverture peut servir à la mesure de différents paramètres de membranes, et que des liquides et/ou échantillons sont ajoutés ou remplacés dans des compartiments quelconques au moyen d'un système de pompage, raccordé par l'intermédiaire de tuyaux flexibles à des compartiments quelconques, ou au moyen d'un procédé à base de pression hydrostatique ou au moyen d'un procédé de piézogouttes et/ou d'un procédé à jet d'encre ou au moyen d'un procédé de transfert à contact ou d'un procédé électro-osmotique ou d'un procédé contrôlé en température.

**11.** Procédé selon l'une des revendications 2 à 10, **caractérisé en ce qu'**il est mis en oeuvre en couplage direct avec un processus de séparation d'échantillons, en particulier électrophorèse capillaire (CE) et HPLC, et sert à l'analyse des substances séparées.

**12.** Procédé selon l'une des revendications 2 à 11, **caractérisé en ce que** le niveau de liquide dans les compartiments est vérifié en continu ou régulièrement et est réajusté en conformité avec des paramètres de remplissage préréglés.

**13.** Procédé selon l'une des revendications 2 à 12, **caractérisé en ce que** la surface (5) du support (1) est structurée de telle sorte qu'il en résulte des zones hydrophiles et hydrophobes, les zones hydrophiles se situant de préférence autour de l'ouverture.

Figur 1

A

B

C

D

Figur 2

Figur 3

Figur 4

Figur 5

Figur 6

figur 7

24

Figur 8

EP 1 040 349 B2

Figur 9A

Figur 9B

Figur 10

EP 1 040 349 B2

Figur 11

Figur 12

10 pA

500 msec

Figur 13A

10 pA

500 msec

Figur 13B

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 290397 A
- US 4055799 A
- EP 0094193 A
- WO 8502201 A

**In der Beschreibung aufgeführte Nicht-Patentliteratur**

- **J.-P. CHANGEUX.** Chemical signalling in the brain. *Sci. Am. Nov.,* 1993, 30 ff
- **A.G. GILMAN.** *Angew. Chem. Int. Ed. Engl.,* 1995, vol. 34, 1406-1428
- **M. RODBELL.** *Angew. Chem. Int. Ed. Engl.,* 1995, vol. 34, 1420-1428
- **J. HODGSON.** *Bio/Technology,* 1992, vol. 9, 973
- **J. KNOWLES.** Medicines for the new millennium hunting down deseases. *Odyssey,* 1997, vol. 3 (1
- **O.P. HAMILL ; A. MARTY et al.** Improved patch-clamp techniques for high-resolution current recording from cells and cell-free membrane patches. *Pflugers Arch,* 1981, vol. 391 (2), 85-100
- **D. MAZIA ; G. SCHATTEN et al.** Adhesion of cells to surfaces coated with polylysine. *J. Cell. biol.,* 1975, vol. 66, 198-200
- **RADLER, J. ; H. STREY et al.** Phenomenology and Kinetics Of Lipid Bilayer Spreading On Hydrophilic Surfaces. *Langmuir,* 1995, vol. 11 (11), 4539-4548
- **J. EDELSTEIN ; O. SCHAAD ; J.-P. CHANGEUX.** Single Binding versus Single Channel Recordings: A new Approach to Study Ionotropic Receptors. *Biochemistry,* 1997, vol. 36, 13755-13760
- **NORTH, R. A.** Ligand- and voltage-gated ion channels. CRC Press, 1994
- **PEROUTKA, S. J.** Serotonin receptor subtypes - Basic and clinical aspects. John Wiley & Sons, 1991
- **PEROUTKA, S. J.** G Protein coupled receptors. CRC Press, 1994
- **CONLEY, E. C.** The ion channel facts book. Academic Press, 1996
- **R.CORONADO ; R. LATORRE.** Phospholipid bilayers made from monolayers on patch-clamp pipettes. *Biophys. J.,* 1983, vol. 43 (2), 231-6
- **D.P. NIKOLELIS ; C.G. SIONTOROU.** Bilayer lipid membranes for flow injection monitoring of acetylcholine, urea, and penicillin. *Anal. Chem.,* 1995, vol. 67 (5), 936-44
- **T.D. OSBORN ; P. YAGER.** Formation of planar solvent-free phospholipid bilayers by langmuir-blodgett transfer of monolayers to micromachined apertures in silicon. *Langmuir,* 1995, vol. 11 (1), 8-12
- **T.D. OSBORN ; P. YAGER.** Modeling success and failure of Langmuir-Blodgett transfer of phospholipid bilayers to silicon dioxide. *Biophys. J.,* 1995, vol. 68 (4), 1364-73
- **P. NOLLERT ; H. KIEFER et al.** Lipid vesicle adsorption versus formation of planar bilayers on solid surfaces. *Biophys. J.,* 1995, vol. 69 (4), 1447-55
- **J. RADLER ; H. STREY et al.** Phenomenology and Kinetics Of Lipid Bilayer Spreading On Hydrophilic Surfaces. *Langmuir,* 1995, vol. 11 (11), 4539-4548
- **M. ERAY ; N.S. DOGAN et al.** A highly stable and selective biosensor using modified nicotinic acetylcholine receptor (nAChR). *Biosystems,* 1995, vol. 35 (2-3), 183-8
- **B. SAKMANN ; E. NEHER.** Single-channel recording. Plenum Press, 1983
- **M.L. WILLIAMSON ; D.H. ATHA et al.** Anti-T2 monoclonal antibody immobilization on quartz fibers: stability and recognition of T2 mycotoxin. *Analytical Letters,* 1989, vol. 22 (4), 803-816
- **H.H. HUB ; U. ZIMMERMANN et al.** Preparation of large unilamellar vesicles. *FEBS Lett.,* 1982, vol. 140 (2), 254-256
- **P. MUELLER ; T.F. CHIEN et al.** Formation and properties of cell-size lipid bilayer vesicles. *Biophys. J.,* 1983, vol. 44 (3), 375-81
- **K. AKASHI ; H. MIYATA et al.** Preparation of giant liposomes in physiological conditions and their characterization under an optical microscope. *Biophys. J.,* 1996, vol. 71 (6), 3242-50
- **M. CRIADO ; B.U. KELLER.** A membrane fusion strategy for single-channel-recordings of membranes usually non-accessible to patch-clamp pipette electrodes. *FEBS Lett.,* 1987, vol. 224 (1), 172-6
- **B.U. KELLER ; R. HEDRICH et al.** Single channel recordings of reconstituted ion channel proteins: an improved technique. *Pflugers Arch,* 1988, vol. 411 (1), 94-100

- **R. HORN ; A. MARTY.** Muscarinic activation of ionic currents measured by a new whole-cell recording method. *J. Gen. Physiol.,* 1988, vol. 92 (2), 145-59
- **J. RAE ; K. COOPER et al.** Low access resistance perforated patch recordings using amphotericin B. *J. Neurosci. Methods.,* 1991, vol. 37 (1), 15-26
- **Z.M. PEI ; J.M. WARD et al.** A novel chloride channel in Vicia faba guard cell vacuoles activated by the serine/threonine kinase, CDPK. *EMBO J.,* 1996, vol. 15 (23), 6564-74
- **R.B. GENNIS.** Biomembranes: molecular structcure and function. Springer Verlag, 1989
- **R.B. GENNIS.** Biomembranes: molecular structure and function. Springer Verlag, 1989
- **SCHÜRHOLZ, T. ; J. KEHNE et al.** Functional reconstitution of the nicotinic acetylcholine receptor by CHAPS dialysis depends on the concentrations of salt, lipid, and protein. *Biochemistry,* 1992, vol. 31 (21), 5067-77
- **SCHÜRHOLZ, T.** Critical dependence of the solubilization of lipid vesicles by the detergent CHAPS on the lipid composition. Functional reconstitution of the nicotinic acetylcholine receptor into preformed vesicles above the critical micellization concentration. *Biophys Chem,* 1996, vol. 58 (1-2), 87-96